(19) Europäisches Patentamt
European Patent Office
Office européen des brevets

(11) EP 4 428 532 A1

(12) EUROPEAN PATENT APPLICATION
published in accordance with Art. 153(4) EPC

(43) Date of publication:
**11.09.2024 Bulletin 2024/37**

(21) Application number: **22906977.8**

(22) Date of filing: **06.10.2022**

(51) International Patent Classification (IPC):
***G01N 33/52*** *(2006.01)* ***C12Q 1/32*** *(2006.01)*
***G01N 21/78*** *(2006.01)*

(52) Cooperative Patent Classification (CPC):
**C12Q 1/32; G01N 21/78; G01N 33/52**

(86) International application number:
**PCT/JP2022/037473**

(87) International publication number:
**WO 2023/112442 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.12.2021 JP 2021201646**

(71) Applicant: **Terumo Kabushiki Kaisha**
**Tokyo 151-0072 (JP)**

(72) Inventor: **AIKAWA, Ryokei**
**Ashigarakami-gun, Kanagawa 259-0151 (JP)**

(74) Representative: **TBK**
**Bavariaring 4-6**
**80336 München (DE)**

(54) **BIOLOGICAL COMPONENT CONCENTRATION MEASUREMENT REAGENT, MEASUREMENT METHOD, AND SENSOR**

(57) Provided is a means capable of quickly measuring a whole blood component concentration even for a hemolyzed whole blood sample. A biological component concentration measuring reagent contains a color-developing pigment, an oxidoreductase, a hemolytic agent, and a low-molecular salt compound, and further contains sucralose.

FIG. 1

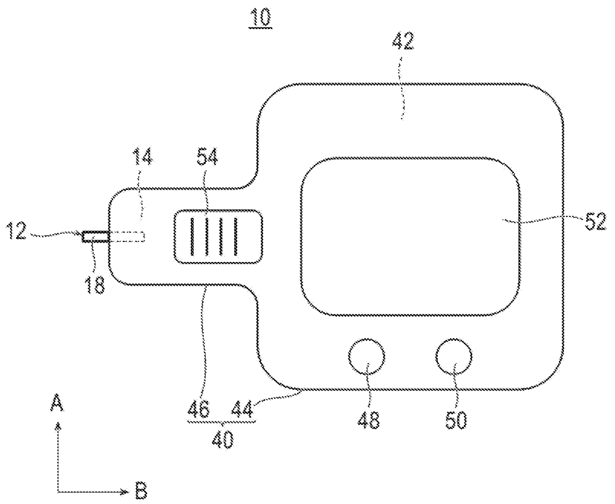




Processed by Luminess, 75001 PARIS (FR)

## Description

### Technical Field

The present invention relates to a biological component concentration measuring reagent, a biological component concentration measuring method, and a sensor. In particular, the present invention relates to a biological component concentration measuring reagent, a biological component concentration measuring method, and a sensor, used for measuring a concentration of an analyte such as glucose contained in a whole blood sample.

### Background Art

Conventionally, in a clinical chemical test, a technique for measuring an analyte (for example, glucose) contained in blood by an electrochemical means or an optical means (colorimetric method) is known.

When an analyte is measured from blood, it is complicated to separate a plasma component, and a color-developing reagent capable of quantifying the analyte with sufficient sensitivity as a whole blood sample is required. However, when measurement is performed using a whole blood sample, hemoglobin reduces a color-developing pigment in a reagent, which may cause interference with the measurement. Therefore, for example, in JP 2000-093198 A (corresponding US Patent No. 5902731), a reagent contains a nitrite.

### Summary of Invention

The above-described biological component measuring reagent is often used as a solid reagent (dry reagent) obtained by applying a reagent liquid and then drying the reagent liquid for incorporation into a sensor or the like.

However, when the dry reagent contains a low-molecular salt compound such as nitrite, there is a problem in terms of accelerating a detection reaction.

Therefore, the present invention has been made in view of the above circumstances, and an object thereof is to provide a means capable of quickly measuring a biological component concentration even for a hemolyzed whole blood sample.

As a result of intensive studies to solve the above problem, the present invention has found that the above problem can be solved by using sucralose in combination even in the presence of a low-molecular salt compound, and has completed the present invention.

That is, the above object can be achieved by the following.

1. A biological component concentration measuring reagent containing a color-developing pigment, an oxidoreductase, a hemolytic agent, and a low-molecular salt compound, and further containing sucralose.
2. The biological component concentration measuring reagent according to the item 1, in which the salt compound is at least one selected from the group consisting of a transition metal salt and an inorganic salt.
3. The biological component concentration measuring reagent according to the item 2, in which the inorganic salt is a nitrite.
4. The biological component concentration measuring reagent according to any one of the items 1 to 3, in which a content mass ratio of the sucralose to the low-molecular salt compound is 1 to 10.
5. The biological component concentration measuring reagent according to any one of the items 1 to 4, further containing a sugar alcohol.
6. The biological component concentration measuring reagent according to the item 5, in which the sugar alcohol is at least one selected from the group consisting of lactitol, erythritol, sorbitol, xylitol, and mannitol.
7. The biological component concentration measuring reagent according to the item 5 or 6, in which a molar ratio of the sugar alcohol to the sucralose is 10% or more.
8. A biological component concentration measuring method including: bringing a whole blood sample into contact with the biological component concentration measuring reagent according to any one of the items 1 to 7; measuring a color development amount; and quantifying a concentration of a biological component in the whole blood sample on the basis of the color development amount.
9. The method according to the item 8, in which the biological component is glucose, cholesterol, neutral lipid, nicotinamide adenine dinucleotide phosphate (NADPH), nicotinamide adenine dinucleotide (NADH), or uric acid.
10. A sensor for measuring a biological component concentration in a whole blood sample, the sensor having a reaction portion, in which the reaction portion includes the biological component concentration measuring reagent according to any one of the items 1 to 7.

Brief Description of Drawings

Fig. 1 is a plan view schematically illustrating a blood sugar meter (component measurement device) to which a measurement sensor according to the present embodiment is attached.
Fig. 2 is an enlarged perspective view illustrating the sensor in Fig. 1 and a photometric unit of a device main body.
Fig. 3 is a top view illustrating the measurement sensor in Fig. 1.
Fig. 4A is a first plan view illustrating an attachment operation of the measurement sensor and the device main body in Fig. 1.
Fig. 4B is a second plan cross-sectional view illustrating an attachment operation subsequent to Fig. 4A.
Fig. 5A is a schematic diagram of a blood sugar meter sensor used in Examples.
Fig. 5B is a diagram for explaining the length, width, and thickness of an inner surface of the blood sugar meter sensor in Fig. 5A.
Fig. 6A is a photograph illustrating an image of a dry reagent and a color-developing image.
Fig. 6B is a graph illustrating a change in color development absorbance with time.
Fig. 7 is a graph illustrating a relationship between a glucose concentration and an absorbance of a chelate compound of formazan and $Zn^{2+}$ when a glucose aqueous solution is caused to act on a blood sugar meter sensor (blood sugar level measuring sensor) to which a tetrazolium salt 1 is applied.

Description of Embodiments

Hereinafter, embodiments of the present disclosure will be described. Note that the present disclosure is not limited only to the following embodiments.

A first aspect of the present disclosure is a biological component concentration measuring reagent containing a color-developing pigment, an oxidoreductase, a hemolytic agent, and a low-molecular salt compound, and further containing sucralose.

A second aspect of the present disclosure is a biological component concentration measuring method including: bringing a whole blood sample into contact with the biological component concentration measuring reagent according to the first aspect of the present disclosure; measuring a color development amount; and quantifying a concentration of a biological component in the whole blood sample on the basis of the color development amount.

A third aspect of the present disclosure is a sensor for measuring a biological component concentration in a whole blood sample, the sensor having a reaction portion, in which the reaction portion includes the biological component concentration measuring reagent according to the first aspect of the present disclosure.

According to the present disclosure, a concentration of a biological component (analyte) (that is, contained in both blood cells and plasma) can be quickly measured for a hemolyzed whole blood sample.

In the present specification, the biological component concentration measuring reagent is also simply referred to as "reagent" or "reagent of the present disclosure".

Hereinafter, embodiments of the present invention will be described. Note that the present invention is not limited only to the following embodiments. In addition, dimensional ratios in the drawings are exaggerated for convenience of description, and may be different from actual ratios.

In addition, in the present specification, a range from "X to Y" includes X and Y, and indicates "X or more and Y or less". "M" means mol/L. In addition, unless otherwise specified, operations and measurements of physical properties and the like are performed at room temperature (20 to 25°C) and at relative humidity of 40 to 50% RH.

<Biological component concentration measuring reagent>

A first aspect of the present disclosure is a biological component concentration measuring reagent containing a color-developing pigment, an oxidoreductase, a hemolytic agent, and a low-molecular salt compound, and further containing sucralose. Hereinafter, each component constituting the reagent will be described in detail.

(Low-molecular salt compound)

The low-molecular compound specifically refers to a compound having a molecular weight of less than 1000, and preferably has a molecular weight of less than 500, more preferably less than 300. A lower limit of the molecular weight of the low-molecular salt compound is not particularly limited, but is usually 30 or more. Note that the low-molecular salt compound does not include a color-developing pigment. In addition, when the salt compound is a hydrate, the compound itself excluding water is defined as the salt compound. That is, when the molecular weight is calculated, a

molecular weight excluding a molecular weight of water is defined as the molecular weight of the salt compound, and the content of the salt compound excluding the content of water is defined as the content of the salt compound.

A preferable aspect of the low-molecular salt compound is at least one selected from the group consisting of a transition metal salt and an inorganic salt. The low-molecular salt compound may be used singly or in combination of two or more types thereof.

When the reagent of the present disclosure contains a tetrazolium salt of the following formula (1) as a color-developing pigment, formazan generated by a reduction reaction of the tetrazolium salt has a maximum absorption wavelength on a long wavelength side. In particular, the tetrazolium salt of the following formula (1) can further shift the maximum absorption wavelength to a longer wavelength side by generating a chelate compound with a transition metal ion. Therefore, the reagent of the present disclosure preferably contains a transition metal salt. That is, in a preferred embodiment of the present invention, the reagent further contains a transition metal salt. In a more preferred embodiment of the present invention, the biological component concentration measuring reagent contains a tetrazolium salt (particularly, the tetrazolium salt of the following formula (1)) as a color-developing pigment, and further contains a transition metal salt. In a still more preferred embodiment of the present invention, the biological component concentration measuring reagent contains a tetrazolium salt (particularly, the tetrazolium salt of the following formula (1)) as a color-developing pigment, and further contains an oxidoreductase and a transition metal salt. According to this embodiment, even when a biological component concentration in a whole blood sample is measured, formazan has a maximum absorption wavelength in a wavelength range (600 nm or more) that does not overlap with a main absorption band of hemoglobin. Therefore, measurement sensitivity of the biological component concentration can be further improved for a biological sample, particularly even for a whole blood sample.

The transition metal salt (also referred to as a transition metal compound) is not particularly limited. Specifically, a compound capable of generating a transition metal ion such as a nickel ion ($Ni^{2+}$), a cobalt ion ($Co^{2+}$), a zinc ion ($Zn^{2+}$), or a copper ion ($Cu^{2+}$) can be used. Such an ion can shift the maximum absorption wavelength of formazan to a longer wavelength side. Among these, a compound capable of generating a nickel ion or a zinc ion is preferable as the transition metal salt. In particular, a zinc ion is more preferable because a time-dependent structural change hardly occurs when the zinc ion reacts with a tetrazolium salt. Therefore, a reagent containing a compound having a zinc ion as a transition metal compound is less likely to be affected by a change in wavelength characteristics due to a temporal structural change, and therefore is less likely to change color-developing characteristics during a reaction and after completion of the reaction. That is, according to a preferred embodiment of the present invention, the transition metal salt is a zinc salt. In addition, the compound that generates the transition metal ion is not particularly limited, but is preferably a compound that generates an ion in an aqueous liquid (for example, water, a buffer solution, blood, or a body fluid). Examples thereof include transition metal salts such as a chloride, a bromide, a sulfate, and an acetate of the above transition metal. Among these, nickel chloride, nickel acetate, zinc chloride, and zinc acetate are preferable, nickel chloride and zinc acetate are more preferable, and zinc acetate is particularly preferable. The transition metal salt may be used singly or in combination of two or more types thereof. In the present embodiment, the content of the transition metal salt is not particularly limited, but for example, when the color-developing pigment is a tetrazolium salt, the content can be appropriately selected according to a desired maximum absorption wavelength of a formazan compound. Specifically, the content of the transition metal salt is such an amount that the content of the transition metal (transition metal ion) is preferably 0.1 to 10 mol, more preferably 0.5 to 4 mol, and particularly preferably more than 1 mol and less than 3 mol with respect to 1 mol of the tetrazolium salt. Alternatively, in the present embodiment, the content (in terms of solid content) of the transition metal salt is such an amount that the content of the transition metal salt is preferably 5 to 30 parts by mass, more preferably 5 to 15 parts by mass, and particularly preferably more than 5 parts by mass and less than 12 parts by mass with respect to 100 parts by mass of the total amount (solid content) of the reagent. With such an amount, a maximum absorption wavelength of a formazan compound can be shifted to a desired wavelength range. In addition, with such an amount, a change in wavelength characteristics due to a temporal structural change can be further suppressed. The content of the transition metal salt is substantially equal to a ratio of the charge amount of the transition metal salt when the reagent is prepared. In addition, when the reagent contains two or more types of transition metal salts, the content of the transition metal salt means the total amount of the transition metal salts to be blended.

Examples of the inorganic salt include salts of inorganic acids such as hydrochloric acid, nitric acid, nitrous acid, sulfuric acid, sulfurous acid, thiosulfuric acid, phosphonic acid, phosphoric acid, phosphomolybdic acid, phosphotungstic acid, and vanadic acid. Examples of salts of these acids include inorganic salts such as a lithium salt, a sodium salt, a potassium salt, a calcium salt, a magnesium salt, and an ammonium salt. Specific examples of the inorganic salt include sodium nitrate, potassium nitrate, ammonium nitrate, magnesium nitrate, calcium nitrate, sodium nitrite, potassium nitrite, ammonium nitrite, sodium sulfate, potassium sulfate, ammonium sulfate, calcium sulfate, magnesium sulfate, sodium sulfite, potassium sulfite, calcium sulfite, magnesium sulfite, potassium thiosulfate, lithium sulfate, magnesium sulfate, sodium thiosulfate, sodium hydrogen sulfite, sodium hydrogen sulfate, potassium hydrogen sulfate, disodium glutarate, lithium fluoride, sodium fluoride, potassium fluoride, calcium fluoride, ammonium fluoride, potassium chloride, sodium chloride, ammonium chloride, calcium chloride, potassium bromide, sodium bromide, ammonium bromide, calcium bromide, sodium iodide, potassium iodide, potassium triiodide, calcium iodide, trilithium phosphate, tripotassium phosphate, trisodium phosphate, triammonium phosphate, sodium monohydrogen phosphate, potassium monohydrogen phosphate, sodium dihydrogen phosphate, potassium dihydrogen phosphate, and ammonium dihydrogen phosphate.

A preferred aspect of the inorganic salt is a nitrite. When a hemolyzed whole blood sample is targeted, hemoglobin (Fe (II)) is released from red blood cells. For example, when a tetrazolium salt is used as a color-developing pigment, hemoglobin non-enzymatically reduces the tetrazolium salt to generate colored formazan, whereby false color development occurs. However, the nitrite that can be contained in the reagent of the present disclosure acts as a methemoglobin-forming agent that oxidizes a divalent iron ion ($Fe^{2+}$) forming a nucleus in hemoglobin to a trivalent iron ion ($Fe^{3+}$). For this reason, even when a hemolyzed whole blood sample is to be measured, nitrous acid takes electrons prior to the color-developing pigment with respect to hemoglobin released from red blood cells, and suppresses non-enzymatic reduction (false color development) of the color-developing pigment.

Examples of the nitrite include sodium nitrite, potassium nitrite, calcium nitrite., and ammonium nitrite. Sodium nitrite and potassium nitrite are preferable, and sodium nitrite is more preferable from a viewpoint of further improving the effect of the present invention, high stability, and high versatility. The nitrite may be used singly or in combination of two or more types thereof.

The content of the nitrite in the reagent is, for example, 0.8 to 10.0 parts by mass, preferably 1.5 to 7.5 parts by mass, and more preferably 2.0 parts by mass or more and less than 5.0 parts by mass with respect to 100 parts by mass of the total amount (solid content) of the reagent. With such an amount, false color development can be more effectively suppressed. Therefore, an analyte concentration (for example, a glucose concentration) can be measured with higher accuracy even for a hemolyzed whole blood sample. In addition, the analyte concentration (for example, a glucose concentration) in the whole blood sample can be measured with higher sensitivity. The content of the nitrite is substantially equal to a ratio of the charge amount of the nitrite when the reagent is prepared. In addition, when the reagent contains two or more types of nitrites, the content of the nitrite means the total amount of the nitrites to be blended.

In addition, the content of the nitrite is preferably 50 mol or less with respect to 1 mol of the color-developing pigment. That is, in a preferred embodiment of the present invention, the nitrite is contained at a ratio of 50 mol or less with respect to 1 mol of the color-developing pigment. When the content of the nitrite with respect to 1 mol of the color-developing pigment is more than 50 mol, the nitrite competes with the color-developing pigment, and therefore a reaction between the color-developing pigment and an enzyme is inhibited (color development inhibition). In particular, when the color-developing pigment is a tetrazolium salt and the reagent further contains an oxidoreductase, the nitrite takes electrons from formazan generated from the tetrazolium salt, and a color development amount corresponding to an object to be measured cannot be obtained. In other words, measurement accuracy may be deteriorated. The content of the nitrite (in terms of the content of nitrite ions ($NO_2^-$)) is more preferably less than 10 mol, still more preferably less than 5 mol, and particularly preferably less than 3.0 mol with respect to 1 mol of the color-developing pigment.

In addition, the content of the nitrite is preferably 0.2 mol or more, more preferably more than 0.5 mol, and particularly preferably more than 1.0 mol with respect to 1 mol of the color-developing pigment. Within such a range, non-enzymatic color development and color development inhibition can be more effectively suppressed. In addition, a sufficient color development amount by the color-developing pigment for measuring an object to be measured can be ensured. In particular, when the color-developing pigment is a tetrazolium salt and the reagent further contains an oxidoreductase (for example, glucose dehydrogenase), the color-developing pigment can take electrons more preferentially than the nitrite with respect to the oxidoreductase, and therefore color development inhibition by nitrous acid (salt) is suppressed (a color development reaction can be more efficiently advanced). Therefore, an analyte concentration can be measured with higher accuracy even for a hemolyzed whole blood sample. That is, according to a preferred embodiment of the present disclosure, nitrous acid or a salt thereof is contained at a ratio of 0.2 mol or more and less than 10 mol with respect to 1 mol of the color-developing pigment. According to a more preferred embodiment of the present disclosure, the nitrite is contained at a ratio of more than 0.5 mol and less than 5 mol with respect to 1 mol of the color-developing pigment. According to a particularly preferred embodiment of the present disclosure, nitrous acid or a salt thereof is contained at a ratio of more than 1.0 mol and less than 3.0 mol with respect to 1 mol of the color-developing pigment. The content of the nitrite is substantially equal to a ratio of the total charge amount (mol) of the nitrites to the total charge amount (mol) of the color-developing pigments when the reagent is prepared. In addition, as the content of the nitrite, a value obtained by dividing the total charge amount (mol) of the nitrites by the total charge amount (mol) of the color-developing pigments when the reagent is prepared, and rounding off the second decimal place of the calculated value to the first decimal place is adopted.

In a preferred embodiment of the present invention, the reagent of the present disclosure contains both a transition metal salt and a nitrite.

(Disaccharide or derivative thereof)

The reagent of the present disclosure contains a disaccharide or a derivative thereof. This can suppress precipitation of a low-molecular salt compound such as a nitrite or a transition metal salt, and can make the reagent transparent. More specifically, the dry reagent can be made amorphous by suppressing precipitation of the salt compound. Dissolution of the reagent in blood can be promoted as compared with a case of not containing the disaccharide or a derivative thereof. Therefore, even when a salt compound is contained, a concentration of an analyte (biological component) can be measured more quickly.

In a preferred embodiment of the present invention, the biological component concentration measuring reagent contains a tetrazolium salt (particularly, the tetrazolium salt of the following formula (1)) as a color-developing pigment, and further contains at least any one of sucralose (1',4,6'-trichlorogalactosucrose), sucrose, and trehalose. In a particularly preferred embodiment of the present invention, the biological component concentration measuring reagent contains a tetrazolium salt (particularly, the tetrazolium salt of formula (1)) as a color-developing pigment, and further contains sucralose, an oxidoreductase, and a transition metal salt.

In the reagent of the present disclosure, the content of the disaccharide or a derivative thereof is not particularly limited, but can be appropriately selected according to the composition of the reagent and the like. Specifically, the content of the disaccharide or a derivative thereof is such an amount that the content of the disaccharide or a derivative thereof is preferably 10 to 50 parts by mass, more preferably 15 to 45 parts by mass, and particularly preferably more than 15 parts by mass and less than 40 parts by mass with respect to 100 parts by mass of the total amount (solid content) of the reagent. With such an amount, precipitation of the low-molecular salt compound can be more effectively suppressed, and the reagent can be made amorphous. The amorphized reagent has transparency. Therefore, a concentration of an analyte (biological component) can be measured still more quickly.

In addition, a content mass ratio of the disaccharide or a derivative thereof to the low-molecular salt compound is preferably 1 to 10, more preferably more than 1 and 5 or less, and still more preferably 1.5 to 5. With such a content mass ratio, precipitation of the low-molecular salt compound can be more effectively suppressed, and transparency of the reagent can be more effectively achieved (false color development can be further suppressed and prevented). Therefore, a concentration of an analyte (biological component) can be measured with higher accuracy. In addition, the content mass ratio of sucralose to the low-molecular salt compound is preferably 1 to 10, more preferably more than 1 and 5 or less, and still more preferably 1.5 to 5. With such a content mass ratio, precipitation of the low-molecular salt compound can be more effectively suppressed, and transparency of the reagent can be more effectively achieved (false color development can be further suppressed and prevented). Therefore, a concentration of an analyte (biological component) can be measured with higher accuracy.

(Sugar alcohol)

The reagent of the present disclosure preferably contains a sugar alcohol. This can improve wettability. Therefore, since an analyte (biological component) quickly spreads over the entire reaction portion (has good spreadability), the analyte (biological component) can react in the entire reaction portion. Therefore, a concentration of the analyte (biological component) can be measured with higher accuracy. That is, in a preferred embodiment of the present invention, the biological component concentration measuring reagent further contains a sugar alcohol. In a more preferred embodiment of the present invention, the biological component concentration measuring reagent contains a tetrazolium salt (particularly, the tetrazolium salt of the following formula (1)) as the color-developing pigment, and further contains a sugar alcohol. In a particularly preferred embodiment of the present invention, the biological component concentration measuring reagent contains a tetrazolium salt (particularly, the tetrazolium salt of the following formula (1)) as a color-developing pigment, and further contains a sugar alcohol, an oxidoreductase, a transition metal salt, an inorganic salt, and a disaccharide or a derivative thereof. Furthermore, in a particularly preferred embodiment of the present invention, the biological component concentration measuring reagent contains a tetrazolium salt (particularly, the tetrazolium salt of the following formula (1)) as a color-developing pigment, and further contains a sugar alcohol, an oxidoreductase, a transition metal salt, an inorganic salt, and sucralose. Here, examples of the sugar alcohol include lactitol, erythritol, sorbitol, xylitol, mannitol, and maltitol. The sugar alcohol may be used singly or in combination of two or more types thereof. Among these, lactitol, erythritol, sorbitol, xylitol, and mannitol are preferable from a viewpoint of suppressing false color development of the color-developing pigment or the like, erythritol, sorbitol, xylitol, and mannitol are more preferable from a viewpoint of further improving wettability or the like, and sorbitol is particularly preferable. In the present embodiment, the content of the sugar alcohol is not particularly limited, but can be appropriately selected according to the composition of the reagent and the like. Specifically, the content of the sugar alcohol is such an amount that the content of the sugar alcohol is preferably 0.1 to 15 parts by mass, more preferably 0.5 to 15 parts by mass, and particularly preferably more than 1 part by mass and less than 10 parts by mass with respect to 100 parts by mass of the total amount (solid content) of the reagent. With such an amount, wettability of the dry reagent can be more effectively improved. Therefore, an analyte (biological component) more quickly spreads over the dry reagent. Therefore, since the analyte (biological component) can more reliably react with the entire dry reagent, a concentration of a target substance can be measured with higher accuracy. Note that the sugar alcohol referred to herein does not include sucralose.

When the reagent of the present disclosure contains a disaccharide or a derivative thereof (particularly sucralose) and a sugar alcohol, a molar ratio of the sugar alcohol to the disaccharide or a derivative thereof (particularly sucralose) is preferably 10% or more, and more preferably 14% or more. By containing the disaccharide or a derivative thereof and the sugar alcohol at such a ratio, blood spreadability is further improved. In addition, the molar ratio of the sugar alcohol to the disaccharide or a derivative thereof (particularly sucralose) is preferably 200% or less, and more preferably 150% or less from a viewpoint of false color developability, still more preferably 100% or less from a viewpoint of transparency, further still more preferably 50% or less from a viewpoint of high temperature deterioration, and particularly preferably 30% or less. As the molar ratio of the sugar alcohol to the disaccharide or a derivative thereof (particularly sucralose), a value obtained to the first decimal place using each molar concentration is adopted.

(Hemolytic agent)

The reagent of the present disclosure contains a hemolytic agent. In general, when a glucose concentration in plasma is measured, it is necessary to consider the amount of red blood cells (hematocrit value). Usually, in a case of optical measurement, the amount of red blood cells is determined by the amount of hemoglobin in red blood cells. However, it is difficult to optically and accurately measure the amount of hemoglobin. In addition, the actual amount of red blood cells does not coincide with the amount of hemoglobin in some cases. In addition, due to light confusion caused by red blood cells, a special measurement method may be required for the measurement itself of the amount of red blood cells. Meanwhile, since the reagent of the present disclosure hemolyzes red blood cells, it is not necessary to consider an influence of light scattering by red blood cells particularly when an analyte is glucose. Therefore, a more accurate amount of hemoglobin (hemoglobin value) can be obtained by a simpler method. In addition, usually, an analyte (for example, glucose) is present in red blood cells in about the same amount as that contained in plasma. Therefore, according to the reagent of the present disclosure, the amount of hemoglobin can be corrected by a simpler method, and a concentration of an analyte (biological component) (for example, glucose) in plasma and blood cells (that is, a whole blood sample) can be measured (That is, a concentration of an analyte in a whole blood sample can be found.). In addition, an operation or a device (for example, centrifugation, filtration, or porous membrane) for separating red blood cells from whole blood is not required. Since it is not necessary to separate plasma, the sample amount is small.

The hemolytic agent may be used singly or in combination of two or more types thereof.

Examples of the hemolytic agent include a nonionic surfactant, an amphoteric surfactant, and an anionic surfactant. A nonionic surfactant is preferable from a viewpoint of not inhibiting enzyme activity.

The nonionic surfactant preferably has an HLB value of 11 or more and 15 or less (more preferably, 12 or more and 14 or less) from a viewpoint of a breaking property of a red blood cell membrane or solubility in water. Examples of such a nonionic surfactant include a polyoxyethylene alkyl ether in which the average number of moles of oxyethylene groups added is 1 or more and 150 or less and an alkyl group has 1 or more and 18 or less carbon atoms, and nonylphenyl polyethylene glycol. As such a nonionic surfactant, a synthesized product or a commercially available product may be used. Examples of the commercially available product include: a polyoxyethylene p-t-octylphenyl ether (Triton-based surfactant) such as polyoxyethylene (9) octylphenyl ether (octylphenoxy poly(ethyleneoxy) ethanol or octylphenyl-polyethylene glycol) (manufactured by Sigma-Aldrich Co., Ltd., Nonidet™ P-40), Triton (registered trademark) X-100 (polyoxyethylene (10) octylphenyl ether), or Triton (registered trademark) X-114 (polyoxyethylene (8) octylphenyl ether); a polyoxyethylene sorbitan fatty acid ester such as Tween (registered trademark) 85; Dodecyl-β-D-maltose; Octyl-β-D-glucoside; Nonidet (registered trademark) P-40 (octylphenoxy poly(ethyleneoxy) ethanol) and a substitute for Nonidet (registered trademark) P-40; Tergitol (registered trademark) NP-10 Surfactant (Nonylphenol Ethoxylate); IGEPAL (registered trademark) CA-630 (octylphenoxy poly(ethyleneoxy) ethanol); EMULGEN (registered trademark) 108 (polyoxyethylene lauryl ether) and EMULGEN (registered trademark) 109P; and Brij (registered trademark) 96 polyethylene glycol monooleyl ether (n = about 2).

As the amphoteric surfactant, a synthesized product or a commercially available product may be used. Examples of the commercially available product include CHAPS (3-(3-cholamidepropyl) dimethylammonio-1-propanesulphonate), alkylpolyaminoethylglycine chloride; and sodium dodecyl sulfate.

The composition (content) of the hemolytic agent in the reagent can be appropriately selected according to a sample amount (whole blood amount). The composition (content) (in terms of solid content) of the hemolytic agent in the reagent is, for example, 1 to 10% by volume with respect to 1.0 μL of a whole blood sample. In addition, the content of the hemolytic agent in a sensor shape is 10 to 50% by mass, and preferably 20 to 40% by mass with respect to the total amount (solid content) of the reagent. With such an amount, for example, a sensor that can sufficiently hemolyze a whole blood sample having a hematocrit of 20 to 70% can be obtained. The content of the hemolytic agent is substantially equal to a ratio of the charge amount of the hemolytic agent when the reagent is prepared. In addition, when the reagent contains two or more types of hemolytic agents, the content of the hemolytic agent means the total amount of the hemolytic agents to be blended.

(Color-developing pigment)

The reagent of the present disclosure contains a color-developing pigment.

The color-developing pigment is preferably a tetrazolium salt from a viewpoint of further improving the effect of the present invention, more preferably 2-benzothiazolyl-3-(4-carboxy-2-methoxyphenyl)-5-[4-(2-sulfoethylcarbamoyl) phenyl]-2H-tetrazolium) or a tetrazolium salt described in WO 2018/051822 A, and particularly preferably the tetrazolium salt described in WO 2018/051822 A.

That is, according to a preferred embodiment of the present disclosure, the color-developing pigment is a tetrazolium salt. According to a more preferred embodiment of the present disclosure, the color-developing pigment is at least one selected from the group consisting of 2-benzothiazolyl-3-(4-carboxy-2-methoxyphenyl)-5-[4-(2-sulfoethylcarbamoyl) phenyl]-2H-tetrazolium and the tetrazolium salt represented by the following formula (1). According to a particularly preferred embodiment of the present disclosure, the color-developing pigment is the tetrazolium salt represented by the following formula (1).

[Chemical Formula 1]

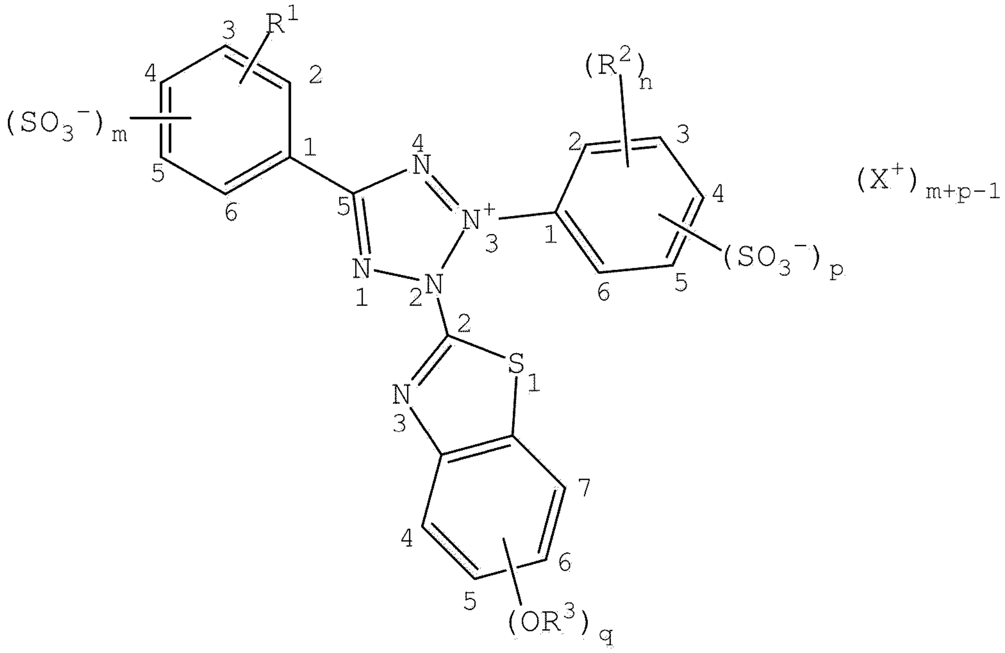

(1)

In the above formula (1), $R^1$ represents any one selected from the group consisting of a hydrogen atom, a hydroxy group, a methoxy group, and an ethoxy group, $R^2$ represents any one selected from the group consisting of a nitro group, $-OR^4$, and a carboxyl group, $R^3$ represents a hydrogen atom, a methyl group, or an ethyl group, in which at least one $R^3$ is a methyl group or an ethyl group, $R^4$ represents a methyl group or an ethyl group, m represents the number of sulfo groups ($-SO_3^-$) bonded to a phenyl group at a 5-position of a tetrazole skeleton, and is 1 or 2, n represents the number of $R^2$s bonded to a phenyl group at a 3-position of the tetrazole skeleton, and is an integer of 0 to 2, p represents the number of sulfo groups ($-SO_3^-$) bonded to a phenyl group at a 3-position of the tetrazole skeleton, and is 0 or 1, n + p is equal to or more than 1, q is 1 or 2, when q is 2, $OR^3$s are disposed adjacent to each other, in which $OR^3$s may form a ring with each other, and X represents a hydrogen atom or an alkali metal.

In formula (1), a substituted benzothiazolyl group is present at a 2-position of the tetrazole skeleton. In the above formula (1), the presence of the benzothiazolyl group at a 2-position of the tetrazole ring makes it possible to efficiently and quickly form a chelate compound with a transition metal compound (a maximum absorption wavelength of a formazan compound can be shifted to a longer wavelength range). When at least one methoxy group or ethoxy group is introduced into the benzothiazolyl group at a 2-position of the tetrazole skeleton, the maximum absorption wavelength at the time of chelation between formazan to be further generated and a transition metal ion such as $Zn^{2+}$ is shifted to a longer wavelength side.

In addition, q is 1 or 2. Preferably, q is 1. Here, when q = 1, $R^3$ is a methyl group or an ethyl group, and is preferably a methyl group from a viewpoint of water solubility. A case where $R^3$ is an alkyl group having 3 or more carbon atoms is not preferable because the tetrazolium salt and formazan generated from the tetrazolium salt are poor in water

solubility.

In formula (1), at least one of $-OR^3$s of the substituted benzothiazolyl group present at a 2-position of the tetrazole skeleton is preferably bonded to a 6-position of the benzothiazolyl group. According to this embodiment, a maximum absorption wavelength at the time of chelation with a transition metal ion such as $Zn^{2+}$ can be shifted to a longer wavelength side.

When q = 1, a substitution position of $-OR^3$ which is a substituent of the benzothiazolyl group present at a 2-position of the tetrazole skeleton is not particularly limited, and may be any one of a 4-position, a 5-position, a 6-position, and a 7-position. The substitution position of $-OR^3$ is preferably a 6-position of the benzothiazolyl group. That is, in a preferred embodiment of the present invention, preferably, q is 1 and $-OR^3$ is bonded to a 6-position of the benzothiazolyl group. In addition, a maximum absorption wavelength at the time of chelation with a transition metal ion such as $Zn^{2+}$ can be shifted to a longer wavelength side.

When q = 2, $R^3$ is a hydrogen atom, a methyl group, or an ethyl group, and at least one of $R^3$s is a methyl group or an ethyl group. In addition, when q is 2, $OR^3$s may be disposed adjacent to each other, and may form a ring with each other. At this time, a suitable combination is a combination in which $R^3$s are a hydrogen atom and a methyl group or a combination in which $R^3$s are a methyl group and a methyl group. When q = 2, substitution positions of $-OR^3$s which are substituent of the benzothiazolyl group present at a 2-position of the tetrazole skeleton are not particularly limited as long as the two $OR^3$s are disposed adjacent to each other, and may be any one of 4,5-positions, 5,6-positions, and 6,7-positions. The substitution position of at least one $-OR^3$ is preferably a 6-position of the benzothiazolyl group, that is, the substitution positions of the two $-OR^3$s are preferably 5,6-positions or 6,7-positions from a viewpoint of an effect of a shift of a maximum absorption wavelength to a longer wavelength side at the time of chelation with a transition metal ion such as $Zn^{2+}$. Specifically, when q is 2, the substituted benzothiazolyl group present at a 2-position of the tetrazole skeleton is preferably any one of the following substituents.

[Chemical Formula 2]

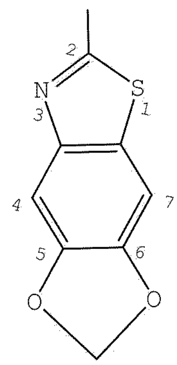

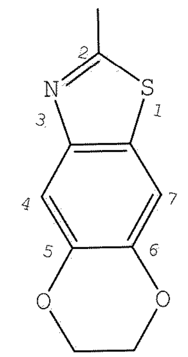

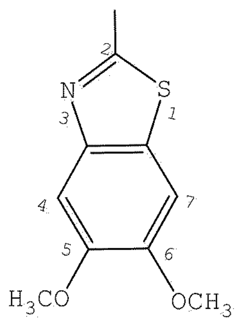

Furthermore, when q is 2, when the substituted benzothiazolyl group present at a 2-position of the tetrazole skeleton is any one of the above substituents, a substituted sulfonated phenyl group present at a 3-position of the tetrazole skeleton is preferably a 4-methoxy-5-sulfophenyl group from a viewpoint of an effect of a shift of a maximum absorption wavelength to a longer wavelength side at the time of chelation with a transition metal ion such as $Zn^{2+}$.

In a preferred embodiment of the present invention, the color-developing pigment is the tetrazolium salt represented by the above formula (1), in which $R^1$ represents any one selected from the group consisting of a hydrogen atom, a hydroxy group, a methoxy group, and an ethoxy group, $R^2$ represents any one selected from the group consisting of a nitro group, $-OR^4$, and a carboxyl group, in which at least one $R^2$ is an $-OR^4$ group, $R^3$ represents a hydrogen atom, a methyl group, or an ethyl group, in which at least one $R^3$ is a methyl group or an ethyl group, $R^4$ represents a methyl group or an ethyl group, m represents the number of sulfo groups ($-SO_3^-$) bonded to a phenyl group at a 5-position of a tetrazole skeleton, and is 1 or 2, n represents the number of $R^2$s bonded to a phenyl group at a 3-position of the tetrazole skeleton, and is 1 or 2, p represents the number of sulfo groups ($-SO_3^-$) bonded to a phenyl group at a 3-position of the tetrazole skeleton, and is 0 or 1, n + p is equal to or more than 1, q is 1 or 2, when q is 2, $OR^3$s are disposed adjacent to each other, in which $OR^3$s may form a ring with each other, and when $OR^3$s form a ring with each other, a substituted benzothiazolyl group present at a 2-position of the tetrazole skeleton is any one of the following substituents:

[Chemical Formula 3]

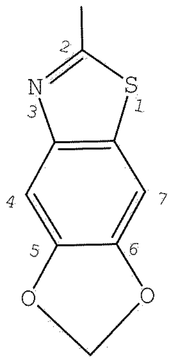
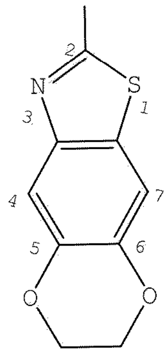

and X represents a hydrogen atom or an alkali metal.

In formula (1), a substituted sulfonated phenyl group is present at a 5-position of the tetrazole skeleton. $R^1$ as a substituent of the sulfonated phenyl group is any one selected from the group consisting of a hydrogen atom, a hydroxy group, a methoxy group, and an ethoxy group. $R^1$ is preferably a hydrogen atom or a hydroxy group from a viewpoint of improving water solubility of the tetrazolium salt and formazan generated from the tetrazolium salt, and $R^1$ is more preferably a hydrogen atom because a chelate with a transition metal ion can be stably formed in a wide pH range. In addition, when $R^1$ is a hydroxy group, a methoxy group, or an ethoxy group, a substitution position thereof is not particularly limited, but is preferably a 4-position.

At a 5-position of the tetrazole skeleton, at least one sulfo group ($-SO_3^-$) is present (m = 1 or 2) . This is considered to improve water solubility of the tetrazolium salt and formazan generated from the tetrazolium salt. In formula (1), m represents the number of sulfo groups ($-SO_3^-$) bonded to the phenyl group at a 5-position of the tetrazole skeleton, and is 1 or 2. In particular, when the sulfo group is present at a 2-position or a 4-position, and furthermore, when the sulfo groups are present at 2,4-positions, water solubility can be further improved. Furthermore, a case where the sulfo groups are present at 2,4-positions is advantageous in that synthesis of a building block for synthesis is easy. Preferably, m = 2, and more preferably, m = 2 and $R^1$ is a hydrogen atom because high water solubility is obtained and a chelate compound with a transition metal ion can be stably formed in a wide pH range, or water solubility is improved.

At this time, when m = 2, p, which is the number of sulfo groups ($-SO_3^-$) bonded to a phenyl group at a 3-position of the tetrazole skeleton, is preferably 1. By selecting the number of substituents, water solubility of the tetrazolium salt and formazan generated from the tetrazolium salt is further improved.

Note that it is preferable to satisfy any one of the following (1) to (4): (1) m = 2 and p = 1, (2) m = 1 and n = 0, (3) in a phenyl group present at a 5-position of the tetrazole skeleton, $R^1$ is a hydroxy group, and at this time, a sulfo group ($SO_3^-$) and the hydroxy group are present at 2,4-positions or 4,6-positions, and (4) p = 0 and at least one $R^2$ is a carboxyl group, and it is more preferable to satisfy (1) m = 2 and p = 1 or (4) p = 0 and at least one $R^2$ is a carboxyl group from a viewpoint of water solubility. In the above (3), it is considered that since the sulfo group ($SO_3^-$) and the hydroxy group are not present as adjacent substituents on the benzene ring, a hydrogen bond is not formed or hardly formed, and both substituents can efficiently contribute to water solubility.

Here, a bonding position of the sulfo group ($-SO_3^-$) to the phenyl group present at a 5-position of the tetrazole skeleton is not particularly limited. When m = 2, the sulfo groups ($-SO_3^-$) are preferably present at 2,4-positions or 3,5-positions of the phenyl group from viewpoints of further improving water solubility of the tetrazolium salt and formazan generated from the tetrazolium salt and being capable of shifting a maximum absorption wavelength to a longer wavelength side. The sulfo groups are particularly preferably present at 2,4-positions of the phenyl group, and a tetrazolium salt compound that is not precipitated even in the presence of a transition metal ion having a high concentration can be formed. That is, by using this tetrazolium salt as a color-developing reagent, a reagent that can be quantified even when an analyte (biological component) has a high concentration can be prepared. That is, in a preferred embodiment of the present invention, the phenyl group at a 5-position of the tetrazole skeleton is preferably a phenyl group in which sulfo groups ($-SO_3^-$) are present at 2,4-positions.

In formula (1), a substituted phenyl group is present at a 3-position of the tetrazole skeleton. Since the phenyl group essentially has a substituent, n + p is 1 or more.

$R^2$ as a substituent of the phenyl group at a 3-position of the tetrazole skeleton is any one selected from the

group consisting of a nitro group, $-OR^4$, and a carboxyl group. $R^2$ is preferably a nitro group or $-OR^4$ from a viewpoint of chelate formability between formazan and a transition metal ion, and is preferably a carboxyl group from a viewpoint of water solubility. In addition, n represents the number of $R^2$s bonded to the phenyl group at a 3-position of the tetrazole skeleton, and is an integer of 0 to 2. By introducing $R^2$, a maximum absorption wavelength of the compound can be shifted to a longer wavelength range, or stability of the compound can be improved, and therefore n is preferably 1 or 2. When two $R^2$s are present, that is, when n = 2, $R^2$s may be the same or different.

When n = 1 or 2, at least one $R^2$ is preferably an $-OR^4$ group. That is, in a preferred embodiment of the present invention, n is 1 or 2, and at least one $R^2$ is an $-OR^4$ group. By introducing an alkoxy group as a substituent of the phenyl group, stability of the compound is improved. The $-OR^4$ group is preferably a methoxy group from a viewpoint of improving water solubility of the tetrazolium salt and formazan generated from the tetrazolium salt. Here, $R^4$ is a methyl group or an ethyl group, and is preferably a methyl group from a viewpoint of water solubility. A case where $R^4$ is an alkyl group having 3 or more carbon atoms is not preferable because the tetrazolium salt and formazan generated from the tetrazolium salt are poor in water solubility.

When n is 1 or 2, a substitution position of $R^2$ is not particularly limited, but is preferably a 2-position, a 3-position, a 4-position, a 5-position, or a 6-position of the phenyl group present at a 3-position of the tetrazole skeleton, at least one $R^2$ is preferably present at a 2-position or a 4-position, and $R^2$s are preferably at a 2-position and/or a 4-position. With such a structure, water solubility is improved, and stability of the tetrazolium salt and formazan generated from the tetrazolium salt can be improved.

p represents the number of sulfo groups ($-SO_3^-$) bonded to the phenyl group at a 3-position of the tetrazole skeleton, and is 0 or 1. p = 1 is preferable from a viewpoint of improving water solubility of the tetrazolium salt and formazan generated from the tetrazolium salt. Note that, when p = 1, since the sulfo group is an electron withdrawing group, when there is another electron withdrawing group (for example, a nitro group), a cationic charge of a nitrogen atom on the tetrazolium ring may be destabilized to lower stability of the compound. As described above, stability of the compound is improved by introducing an alkoxy group as a substituent of a phenyl group, but when a nitro group is simultaneously introduced, the stability is not improved in some cases by introduction of the alkoxy group. Therefore, when p = 1, n is 1 or 2, preferably 1, and $R^2$ is preferably any one selected from the group consisting of $-OR^4$ and a carboxyl group, and more preferably $-OR^4$ from a viewpoint of improving stability. Alternatively, preferably, p = 0 and at least one $R^2$ is a carboxyl group from a viewpoint of improving water solubility of the tetrazolium salt and formazan generated from the tetrazolium salt. That is, in a preferred embodiment, in formula (1), p is 1, or p = 0 and at least one $R^2$ is a carboxyl group. More preferably, m = 2 and p = 1, or p = 0 and at least one $R^2$ is a carboxyl group.

When p = 1, a substitution position of the sulfo group ($-SO_3^-$) as a substituent of the phenyl group at a 3-position of the tetrazole skeleton is not particularly limited, but is preferably at a 3-position or a 5-position. With the sulfo group as a substituent at this position, stability of the tetrazolium salt and formazan generated from the tetrazolium salt can be more effectively improved.

In addition, in formula (1), the substituent present at a 3-position of the tetrazole skeleton is preferably a 4-methoxy-3-sulfophenyl group, a 2-methoxy-5-sulfophenyl group, a 2-methoxy-4-nitro-5-sulfophenyl group, a 2-methoxy-4-nitrophenyl group, a 4-sulfophenyl group, a 4-carboxy-2-methoxyphenyl group, a 5-carboxy-2-methoxyphenyl group, a 3-carboxy-4-methoxyphenyl group, or a 4-methoxy-5-sulfophenyl group, more preferably a 4-methoxy-3-sulfophenyl group, a 2-methoxy-5-sulfophenyl group, a 3-carboxy-4-methoxyphenyl group, or a 4-methoxy-5-sulfophenyl group, and particularly preferably a 4-methoxy-3-sulfophenyl group, a 4-methoxy-5-sulfophenyl group, or a 2-methoxy-5-sulfophenyl group. With such a structure, color development sensitivity is improved, water solubility is improved, and stability of the tetrazolium salt and formazan generated from the tetrazolium salt can be improved. In addition, the phenyl group present at a 3-position of the tetrazole skeleton is particularly preferably a 4-methoxy-3-sulfophenyl group because a maximum absorption wavelength of the formazan compound itself can be shifted to a longer wavelength range.

In the above formula (1), X represents a hydrogen atom or an alkali metal. Here, X is present in order to neutralize an anion (sulfo group ($-SO_3^-$)). Therefore, the type of the alkali metal is not particularly limited, and may be any one of lithium, sodium, potassium, rubidium, and cesium.

Preferred examples of the tetrazolium salt include those having the following structures. In the following structures, X represents an alkali metal.

[Chemical Formula 4]

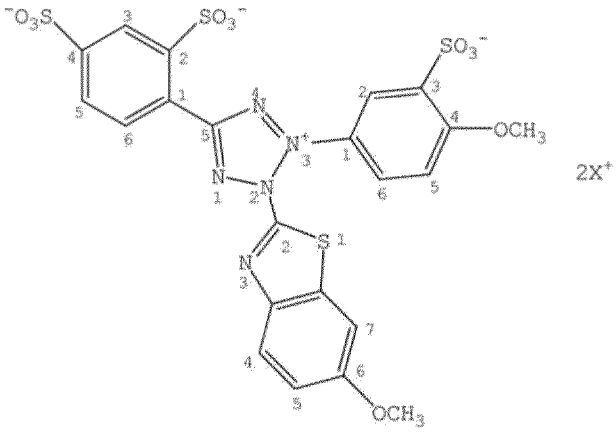

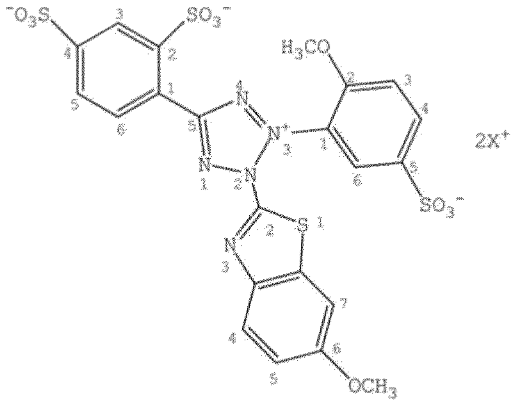

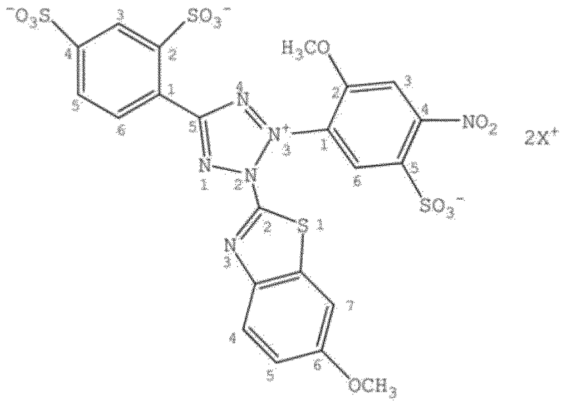

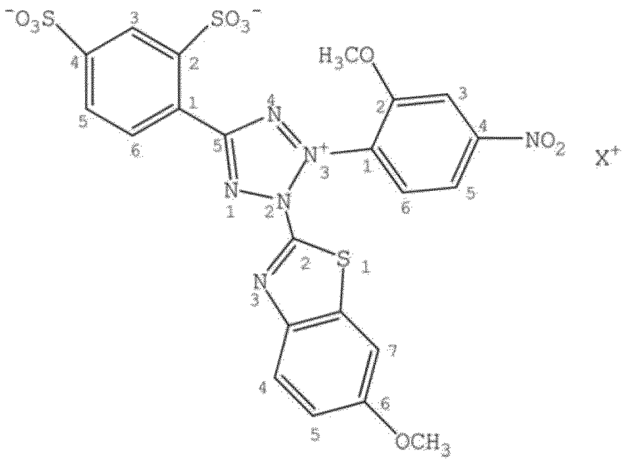

[Chemical Formula 5]

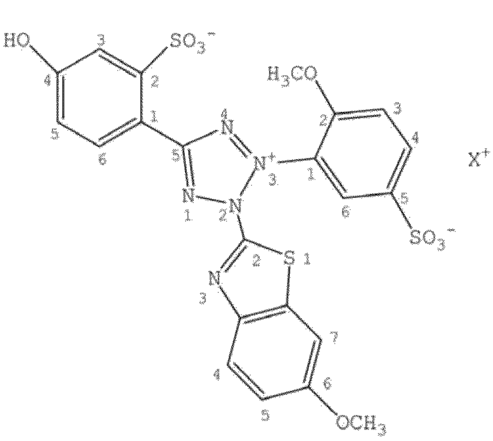

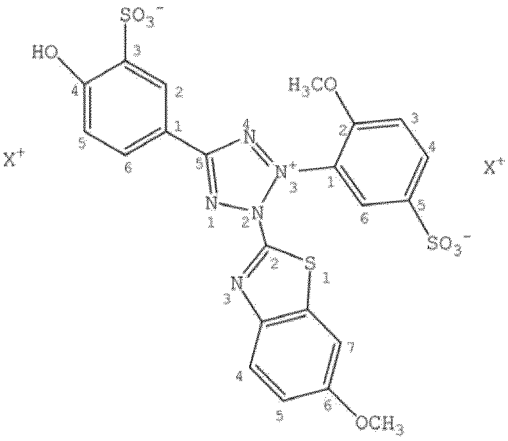

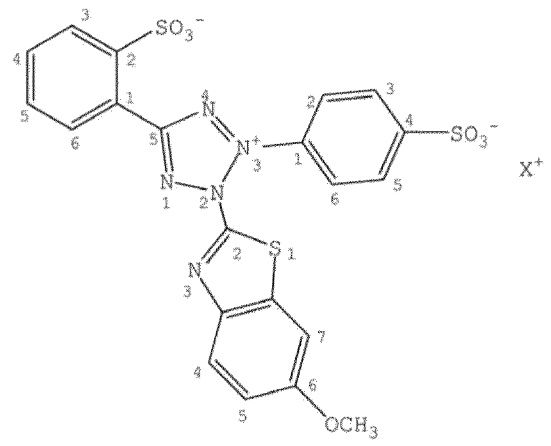

[Chemical Formula 6]

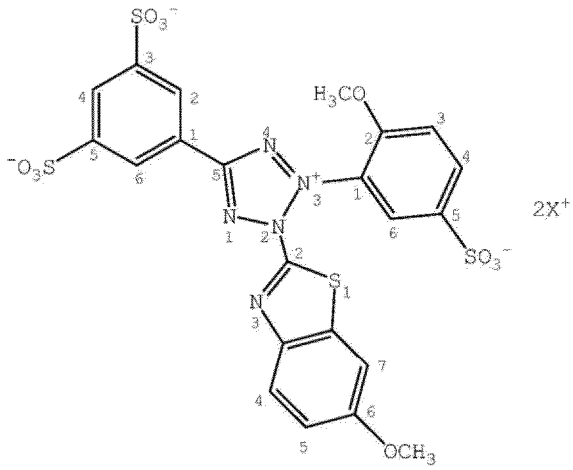

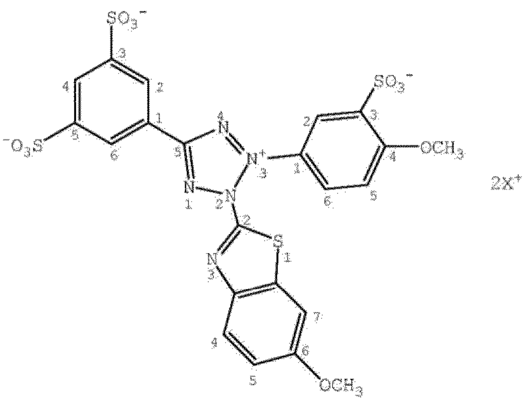

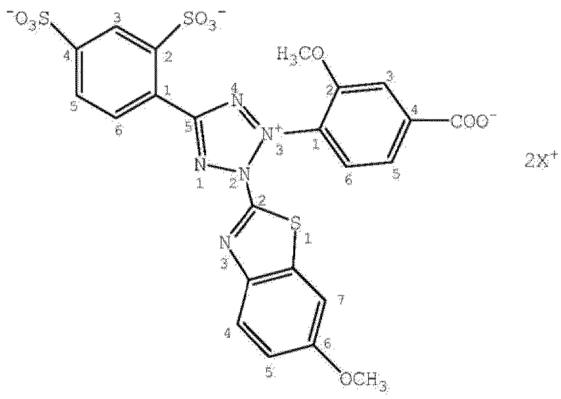

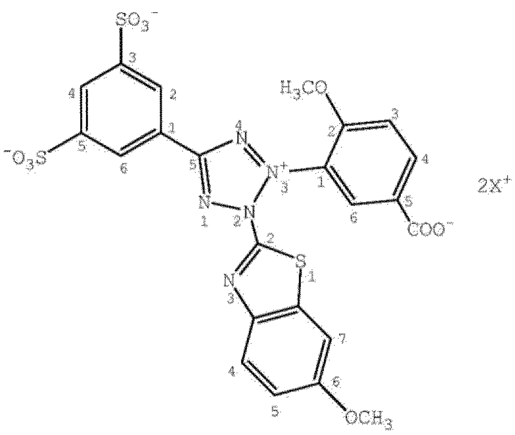

[Chemical Formula 7]

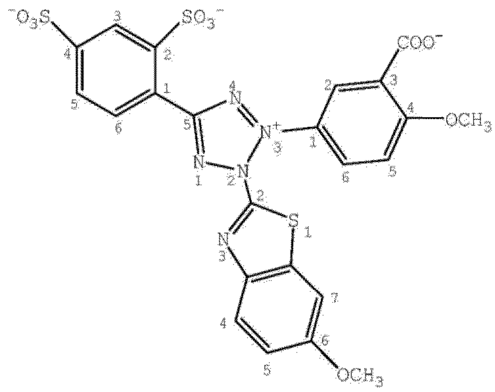

2X+

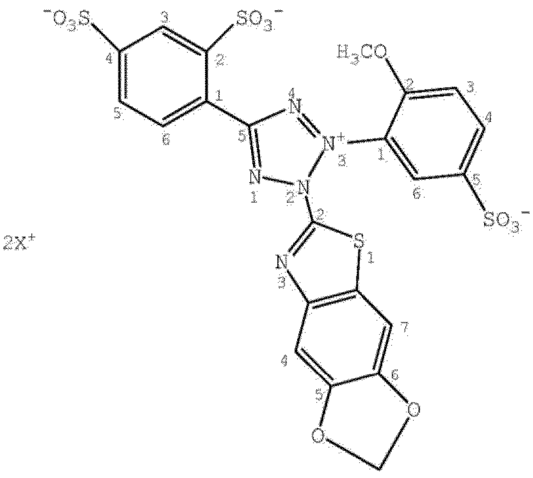

2X+

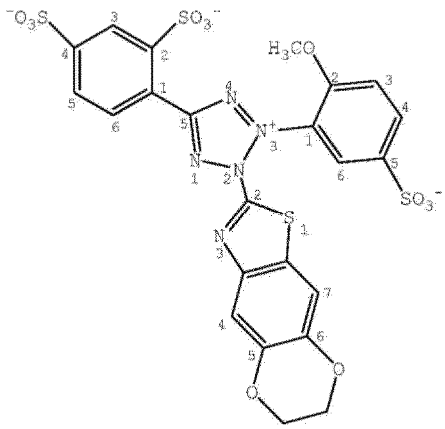

2X+

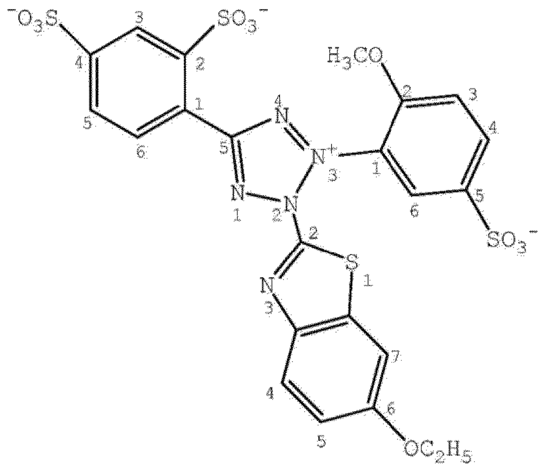

2X+

[Chemical Formula 8]

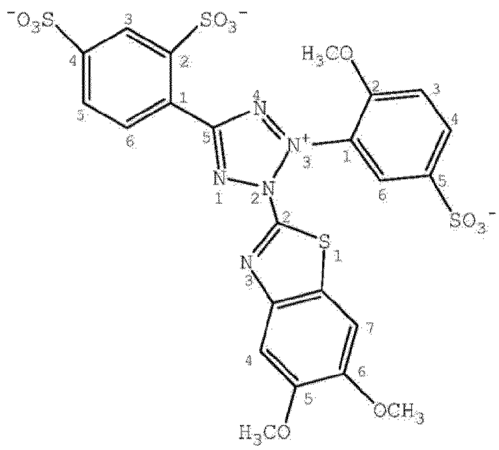

2X+

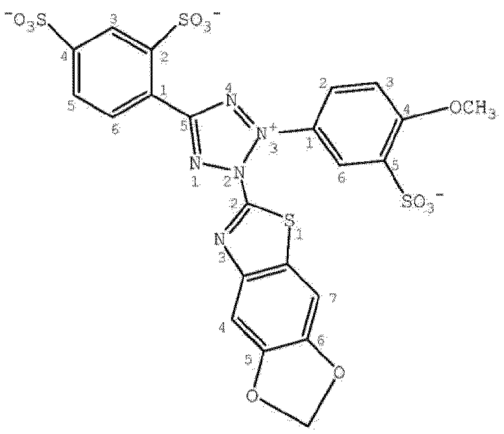

2X+

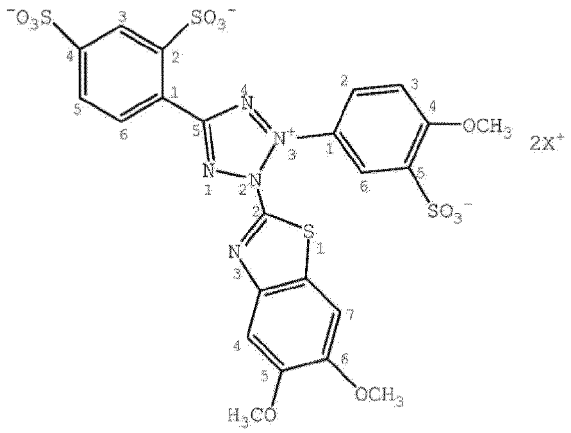

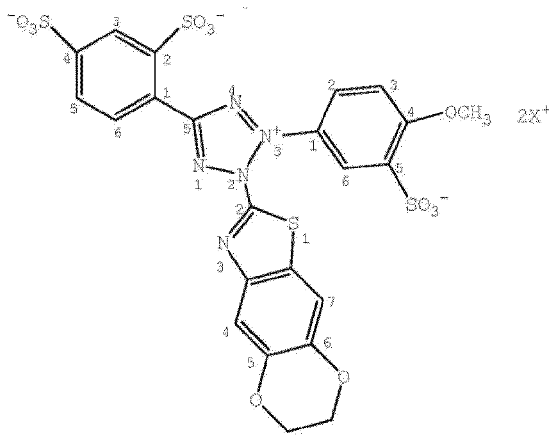

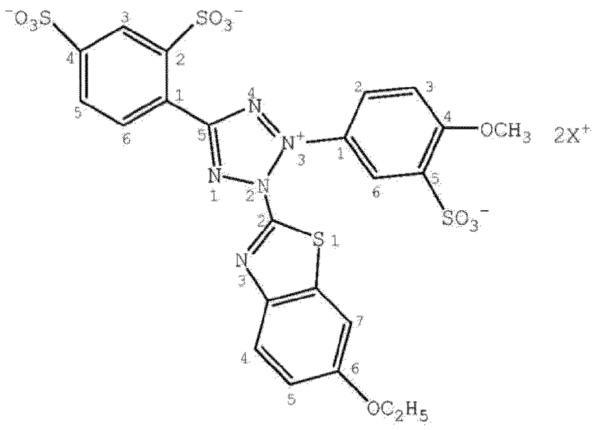

A particularly preferred example of the tetrazolium salt is the following structure. That is, in a preferred embodiment of the present invention, the color-developing pigment has the following structure.

[Chemical Formula 9]

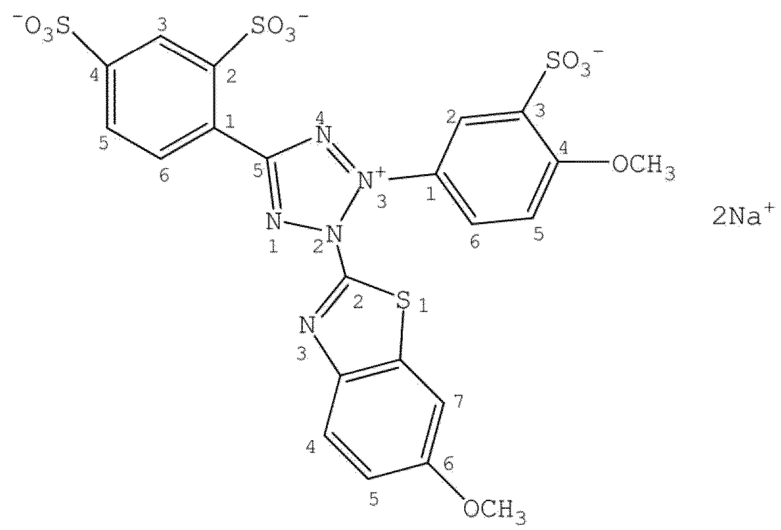

As the color-developing pigment, a synthesized product or a commercially available product may be used. For example, (2-benzothiazolyl-3-(4-carboxy-2-methoxyphenyl)-5-[4-(2-sulfoethylcarbamoyl) phenyl]-2H-tetrazolium) preferably used in the present disclosure is commercially available from Dojindo Molecular Technologies, Inc. under a trade name of WST-4. In addition, the tetrazolium salt of the above formula (1) preferably used in the present disclosure is not limited to the following, but can be manufactured, for example, by a method described in WO 2018/051822 A or a method in which the method is appropriately modified. As an example, hydrazone is synthesized by dehydration condensation of aldehyde and hydrazine, and subsequently, a corresponding diazonium salt is caused to react with hydrazone in an aqueous solvent under basic conditions to obtain formazan. Here, as a basifying agent, sodium hydroxide, potassium hydroxide, or the like is used. Subsequently, the obtained formazan is oxidized in an alcohol solvent (for example, methanol or ethanol) using an oxidizing agent such as ethyl nitrite, butyl nitrite, or sodium hypochlorite to obtain the tetrazolium salt of formula (1). In one embodiment, a hydrazino-substituted benzothiazole having the following structure:

[Chemical Formula 10]

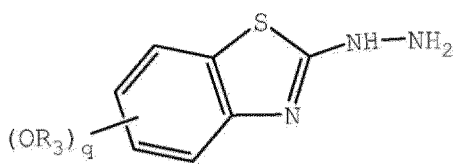

and a substituted sulfonated benzaldehyde having the following structure:

[Chemical Formula 11]

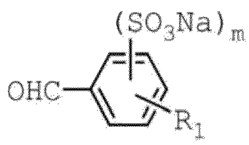

are caused to react with each other to obtain a hydrazone compound having the following structure:

[Chemical Formula 12]

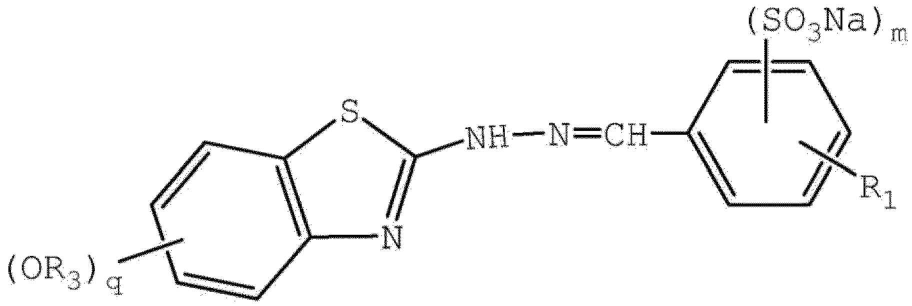

Meanwhile, hydrochloric acid is added to a substituted sulfonated aniline having the following structure:

[Chemical Formula 13]

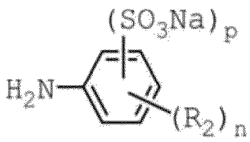

while the substituted sulfonated aniline is cooled with ice, and a sodium nitrite solution is further added dropwise thereto to obtain a benzenediazonium chloride compound having the following structure:

[Chemical Formula 14]

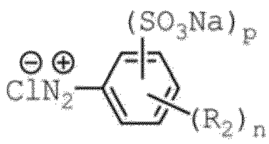

The hydrazone compound and the benzenediazonium chloride compound obtained above were caused to react with each other under basic conditions (for example, in the presence of sodium hydroxide or potassium hydroxide) to obtain a formazan compound having the following structure:

[Chemical Formula 15]

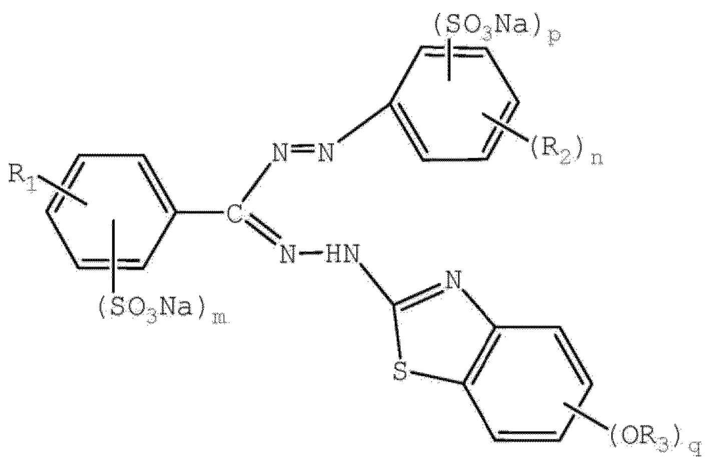

Subsequently, the formazan compound thus obtained is oxidized in an alcohol solvent (for example, methanol or ethanol) using an oxidizing agent (for example, sodium nitrite or a nitrous acid ester such as ethyl nitrite or butyl nitrite) to obtain the tetrazolium salt of the above formula (1).

The composition (content) (in terms of solid content) of the color-developing pigment in the reagent is, for example, 10 to 40 parts by mass, preferably 15 to 35 parts by mass, and more preferably more than 15 parts by mass and less than 30 parts by mass with respect to 100 parts by mass of the total amount (solid content) of the reagent. With such an amount, the color-developing pigment (for example, tetrazolium salts) can exhibit a sufficient color development amount with respect to an analyte. Therefore, an analyte concentration (for example, a glucose concentration) in a hemolysis sample can be measured with higher accuracy. The content of the color-developing pigment is substantially equal to a ratio of the charge amount of the color-developing pigment when the reagent is prepared. In addition, when the reagent contains two or more types of color-developing pigments, the content of the color-developing pigment means the total amount of the color-developing pigments to be blended.

Formazan generated from the tetrazolium salt of the above formula (1) or a chelate compound of formazan and a transition metal ion has a large absorption wavelength band in a wavelength range (600 nm or more) that does not overlap with a main absorption band of hemoglobin singly or by forming a chelate compound with a transition metal compound. In addition, the tetrazolium salt of the above formula (1) has high water solubility. Therefore, by using the tetrazolium salt of the above formula (1), a biological component concentration can be measured with high sensitivity even for a hemolyzed whole blood sample. Specifically, a maximum absorbance (cell length = 0.045 mm in 200 mg/dL glucose aqueous solution) of formazan generated from the tetrazolium salt of the above formula (1) or a chelate compound of formazan and a transition metal ion at a wavelength of 600 nm or more and 800 nm or less is 0.3 or more, and more preferably 0.5 or more. When formazan or a chelate compound of formazan and a transition metal ion (therefore, a tetrazolium salt capable of generating such formazan) having such absorption is used, a biological component concentration is hardly affected by absorption of blood, and can be more accurately measured with good sensitivity. Note that, in the present specification, unless otherwise specified, as a maximum absorption wavelength (λmax) of a chelate compound of formazan and a transition metal ion, a value measured according to the following method is adopted.

-Evaluation of maximum absorption wavelength (Xmax) of chelate compound of formazan and transition metal ion-

A 10 mM MOPS aqueous solution is added such that a final concentration of a formazan compound is 50 to 200 mM to prepare a sample. Separately, an aqueous solution containing 1 M transition metal ions (for example, nickel ions) is prepared.

To 100 μL of the sample, 10 μL of the separately prepared aqueous solution containing transition metal ions is added, and the mixture is quickly stirred to prepare a mixed liquid. A spectrum of the mixed solution is measured with a spectrophotometer (measurement cell length: 10 mm). A maximum absorption wavelength (nm) at 600 nm or more is obtained on the basis of the spectrum. Note that any wavelength can be selected as the maximum absorption wavelength (λmax) as long as the wavelength satisfies a maximum absorbance (0.3 or more) at 600 nm or more.

(Oxidoreductase)

The reagent of the present disclosure contains an oxidoreductase. In a more preferred embodiment of the present invention, the biological component concentration measuring reagent contains a tetrazolium salt (particularly, the tetrazolium salt of the above formula (1)) as a color-developing pigment, and further contains an oxidoreductase that specifically reacts with an analyte.

Here, the oxidoreductase is not particularly limited, and can be appropriately selected depending on the type of biological component to be measured. Specific examples thereof include glucose dehydrogenase (GDH) such as glucose dehydrogenase (GDH), glucose dehydrogenase (PQQ-GDH) using pyrroloquinoline quinone (PQQ) as a coenzyme, glucose dehydrogenase (FAD-GDH) using flavin adenine dinucleotide (FAD) as a coenzyme, glucose dehydrogenase (NAD-GDH) using nicotinamide adenine dinucleotide (NAD) as a coenzyme, or glucose dehydrogenase (NADP-GDH) using nicotinamide adenine dinucleotide phosphate (NADP) as a coenzyme, glucose oxidase (GOD), lactic acid dehydrogenase (LDH), cholesterol dehydrogenase, cholesterol oxidase, and uric acid dehydrogenase. Here, the oxidoreductase may be used singly or in combination of two or more types thereof. For example, when an analyte (biological component) is glucose, the oxidoreductase is preferably glucose dehydrogenase or glucose oxidase. In addition, when the analyte (biological component) is cholesterol, the oxidoreductase is preferably cholesterol dehydrogenase or cholesterol oxidase. When the biological component concentration measuring reagent contains an oxidoreductase, the content of the oxidoreductase is not particularly limited, and can be appropriately selected according to the amount of the tetrazolium salt.

(Other components)

The reagent of the present disclosure essentially contains a color-developing pigment, an oxidoreductase, a hemolytic agent, a low-molecular salt compound, and a disaccharide or a derivative thereof (particularly sucralose). The reagent of the present disclosure may contain another component in addition to the above components. Here, as the other component, usually, a component that is appropriately selected according to the type of a biological component to be measured and added for measuring a biological component concentration can be similarly used. Specific examples thereof include a transition metal compound, an electron carrier, and a pH buffer. Here, each of the above components may be used singly or in combination of two or more types thereof. In addition, each of the above components may be used singly or in combination of two or more types thereof. Note that the reagent of the present disclosure preferably contains no surfactant other than a hemolytic agent.

The electron carrier is not particularly limited, and a known electron carrier may be used. Specific examples thereof include diaphorase, phenazine methosulfate (PMS), 1-methoxy-5-methylphenazinium methylsulfate (1-methoxy PMS or m-PMS), nicotinamide adenine dinucleotide phosphate (NADPH), and nicotinamide adenine dinucleotide (NADH). Here, the electron carrier may be used singly or in combination of two or more types thereof. When the biological component concentration measuring reagent contains an electron carrier, the content of the electron carrier is not particularly limited, and can be appropriately selected according to the amount of the tetrazolium salt.

A use form of the reagent is not particularly limited, and may be any one of a solid form, a gel form, a sol form, and a liquid form. In the reagent of the present disclosure, the reagent is preferably used in a solid (dry state), that is, the reagent of the present disclosure is preferably a dry reagent.

The dry reagent is usually formed by applying a coating liquid for forming a dry reagent to a substrate. The coating liquid may contain water, a buffer (pH buffer), or the like from a viewpoint of formability. Here, the buffer is not particularly limited, and a buffer generally used in measuring a biological component concentration can be used similarly. Specific examples thereof include: a GOOD buffer such as a phosphate buffer, a citrate buffer, a citrate-phosphate buffer, a tris-hydroxymethylaminomethane-HCl buffer (tris-hydrochloride buffer), a MES buffer (2-morpholinoethanesulfonic acid buffer), a TES buffer (N-tris(hydroxymethyl) methyl-2-aminoethanesulfonic acid buffer), an acetate buffer, a MOPS buffer (3-morpholinopropanesulfonic acid buffer), a MOPS-NaOH buffer, a HEPES buffer (4-(2-hydroxyethyl)-1-piperazineethanesulfonic acid buffer), or a HEPES-NaOH buffer; an amino acid-based buffer such as a glycine-hydrochloride buffer, a glycine-NaOH buffer, a glycylglycine-NaOH buffer, or a glycylglycine-KOH buffer; a borate-based buffer such as a tris-borate buffer, a borate-NaOH buffer, or a borate buffer; and an imidazole buffer. Among these, a phosphate buffer, a citrate buffer, a citrate-phosphate buffer, a tris-hydrochloride buffer, a MES buffer, an acetate buffer, a MOPS buffer, and a HEPES-NaOH buffer are preferable. Here, a concentration of the buffer is not particularly limited, but is preferably 0.01 to 1.0 M. Note that, in the present invention, the concentration of the buffer refers to a concentration (M, mol/L) of the buffer contained in a coating liquid. The buffer solution preferably has a pH near a neutral value, for example, a pH of about 5.0 to 8.0. Note that a concentration of the tetrazolium salt in the coating liquid is not particularly limited as long as a desired biological component concentration can be measured with the concentration of the tetrazolium salt, but the tetrazolium salt is preferably contained in a sufficient amount with respect to the abundance of the desired biological component. In consideration of the above viewpoint, a biological component concentration to be usually measured, and the like, the concentration of the tetrazolium salt is preferably 0.01 to 0.2 mol/L, and more preferably 0.05 to 0.1 mol/L in the coating liquid. With such an amount, the tetrazolium salt reacts according to the amount of substantially all (for example, 95 mol% or more, preferably 98 mol% or more, and particularly preferably 100 mol%) biological components contained in the biological sample. Therefore, a desired biological component concentration can be measured accurately and quickly with good sensitivity.

By using the reagent of the present disclosure, a concentration of a specific biological component contained in a whole blood sample can be measured with high accuracy. In addition, by using the reagent of the present disclosure, a concentration of a biological component (analyte) (that is, contained in both blood cells and plasma) can be measured with high accuracy for a hemolyzed whole blood sample.

Therefore, the present invention also provides a biological component concentration measuring method including: bringing a whole blood sample into contact with the biological component concentration measuring reagent of the present disclosure; measuring a color development amount; and quantifying a concentration of a biological component in the whole blood sample on the basis of the color development amount.

In the present disclosure, a sample to be measured is whole blood. A whole blood sample is introduced into a sensor including the biological component concentration measuring reagent of the present disclosure or the pigment or a sensor attached to a component measurement device (for example, a blood sugar meter), the whole blood sample is brought into contact with the reagent, and measurement is thereby performed. In addition, the biological component is not particularly limited, and usually, a biological component to be measured by a colorimetric method or an electrode method can be used similarly. Specific examples thereof include glucose, cholesterol, neutral lipid, nicotinamide adenine dinucleotide phosphate (NADPH), nicotinamide adenine dinucleotide (NADH), and uric acid. That is, according to a

preferred embodiment of the present invention, the reagent of the present disclosure is used for measuring a concentration of glucose, cholesterol, neutral lipid, nicotinamide adenine dinucleotide phosphate (NADPH), nicotinamide adenine dinucleotide (NADH), or uric acid in whole blood. In addition, according to a preferred embodiment of the present invention, the biological component is glucose, cholesterol, neutral lipid, nicotinamide adenine dinucleotide phosphate (NADPH), nicotinamide adenine dinucleotide (NADH), or uric acid.

In the present disclosure, the measurement method is not particularly limited, and can be appropriately selected according to the type of biological component to be measured. For example, when the color-developing pigment is a tetrazolium salt, the biological component is β-D-glucose, and the oxidoreductase is glucose dehydrogenase (GDH), glucose is oxidized by GDH to generate gluconic acid. By using a fact that a coenzyme of GDH or an electron carrier is reduced at this time, specifically, the method is roughly divided into a method (colorimetric method) for optically measuring a color degree of a tetrazolium salt (therefore, formazan or a chelate compound of formazan and a transition metal ion) reduced as a result, and a method (electrode method) for measuring a current generated by an oxidation-reduction reaction. Out of the above methods, measurement of a blood sugar level by the colorimetric method has advantages that it is easy to perform correction using a hematocrit value when calculating the blood sugar level, a manufacturing process is simple, and the like. Therefore, the biological component concentration measuring reagent can be preferably used for the colorimetric method.

In particular, when a glucose concentration in a whole blood sample is measured, the colorimetric method is preferably used.

The reagent of the present disclosure may be used for measuring a biological component concentration in a form in which components are mixed, or may be incorporated into a sensor (sensor for measuring a concentration of a whole blood component) in a dry state. That is, the present invention also provides a sensor for measuring a biological component concentration in a whole blood sample, the sensor having a reaction portion, in which the reaction portion includes the biological component concentration measuring reagent of the present disclosure (hereinafter, also simply referred to as "sensor"). In addition, the reagent and the method of the present disclosure can be incorporated into an automatic analyzer, a measurement kit, a simple blood sugar meter, or the like, and can be used for a daily clinical test. In addition, the reagent of the present disclosure can also be incorporated into a commercially available biosensor. Note that when the reagent of the present disclosure is incorporated into a sensor, the content of the reagent per sensor is not particularly limited, and usually, an amount used in the field can be adopted similarly. However, the color-developing pigment (particularly, the tetrazolium salt of formula (1)) is preferably contained in a sufficient amount with respect to the abundance of a desired biological component. In consideration of the above viewpoint, a biological component concentration to be usually measured, and the like, a concentration of the color-developing pigment is preferably 3 to 50 nmol, and more preferably 10 to 30 nmol per sensor. With such an amount, the color-developing pigment reacts according to the amounts of substantially all biological components contained in the whole blood sample. Therefore, a desired biological component concentration can be measured accurately and quickly with good sensitivity.

Hereinafter, a form of the measurement sensor (colorimetric blood sugar meter) of the present disclosure used to measure a blood sugar level by a colorimetric method will be described with reference to the drawings. However, the present invention is characterized by using the biological component concentration measuring reagent of the present disclosure, and a structure of the sensor is not particularly limited. Therefore, the biological component concentration measuring reagent of the present disclosure may be applied to a commercially available measurement sensor and sensors described in WO 2016/051930 A, WO 2018/51822 A, WO 2020/137532 A, and the like. Similarly, in the following embodiment, a specific form of a sensor for the purpose of measuring a blood sugar level will be described, but the measurement sensor is not limited to the application, and can be similarly applied to other applications or can be appropriately modified and applied.

Fig. 1 is a plan view schematically illustrating a blood sugar meter used for detecting glucose (blood sugar) using the measurement sensor according to the present embodiment.

In Fig. 1, a blood sugar meter 10 is configured as a device for measuring glucose (blood sugar) in a whole blood sample. The blood sugar meter 10 can be mainly used for personal use operated by a user (subject). The user can also measure his or her blood sugar before eating to manage his or her blood sugar. In addition, a medical worker can also use the blood sugar meter 10 in order to evaluate a health condition of a subject, and in this case, the blood sugar meter 10 may be appropriately modified and configured to be installable in a medical facility or the like.

The blood sugar meter 10 adopts a principle of a colorimetric method that optically measures the content (blood sugar level) of glucose contained in a whole blood sample. In particular, the blood sugar meter 10 performs blood sugar measurement by a transmission-type measurement unit 14 that emits measurement light having a predetermined wavelength and receives light that has passed through a measurement area of a measurement sensor 12.

To the blood sugar meter 10, the measurement sensor 12 including blood is attached, or blood is introduced into the measurement sensor 12 while the measurement sensor 12 is attached to the blood sugar meter 10, and the measurement unit 14 detects glucose. The measurement sensor 12 may be configured as a disposable type that is discarded for each measurement. Meanwhile, the blood sugar meter 10 is preferably configured as a portable and robust

device such that a user can easily repeat measurement.

As illustrated in Fig. 2, the measurement sensor 12 includes a sensor main body 18 formed in a plate shape and a cavity 20 (cavity for liquid) extending in a plane direction of a plate surface inside the sensor main body 18.

As illustrated in Fig. 2, the sensor main body 18 is formed in a rectangular shape having a long side 22 in insertion and detachment directions (distal end and proximal end directions of the blood sugar meter 10, that is, direction B) of the blood sugar meter 10 and having a short side 24 in direction A. For example, the length of the long side 22 of the sensor main body 18 is preferably set to twice or more the length of the short side 24. As a result, a sufficient insertion amount of the measurement sensor 12 with respect to the blood sugar meter 10 is ensured.

In addition, the thickness of the sensor main body 18 is formed so as to be extremely small (thin) as compared with a side surface formed in a rectangular shape (In Fig. 2, the sensor main body 18 is illustrated so as to have a sufficient thickness intentionally.). For example, the thickness of the sensor main body 18 is preferably set to 1/10 or less of the short side 24 described above. The thickness of the sensor main body 18 is preferably designed appropriately according to the shape of an insertion hole 58 of the blood sugar meter 10.

In the measurement sensor 12, a pair of plate pieces 30 and a pair of spacers 32 constitute the sensor main body 18 so as to have the cavity 20.

Fig. 3 is a top view illustrating the measurement sensor in Fig. 1. In Fig. 3, a corner of the sensor main body 18 is sharp, but for example, the corner may be formed in a round corner. In addition, the sensor main body 18 is not limited to a thin plate shape, and it is a matter of course that the shape may be freely designed. For example, the sensor main body 18 may be formed in a square shape, another polygonal shape, a circular shape (including an elliptical shape), or the like in top view.

The cavity 20 formed inside the sensor main body 18 is at a midpoint in a short axis direction of the sensor main body 18, and is formed linearly in a longitudinal direction of the sensor main body 18. The cavity 20 is connected to a distal end port 20a formed on a distal end side 24a of the sensor main body 18 and a proximal end port 20b formed on a proximal end side 24b, and communicates with the outside of the sensor main body 18. When blood of a user is taken into the cavity 20 from the distal end port 20a, the cavity 20 can cause the blood to flow in an extending direction on the basis of a capillary phenomenon. The amount of blood flowing through the cavity 20 is small, and leakage is suppressed by tension even when the blood moves to the proximal end port 20b. Note that an absorption portion (for example, a portion obtained by making the spacer 32 described later only on the proximal end side a porous body) that absorbs blood may be disposed on a side of the proximal end side 24b of the sensor main body 18.

In addition, a reagent (color-developing reagent) 26 that exhibits a color corresponding to a concentration of glucose (blood sugar) in blood by reacting with glucose (blood sugar) in blood is applied to a predetermined position (for example, a position slightly closer to the proximal end than a midpoint between the distal end port 20a and the proximal end port 20b illustrated in Fig. 3) of the cavity 20, and a measurement target portion 28 to be measured by the blood sugar meter 10 is set. Blood flowing in the proximal end direction through the cavity 20 comes into contact with the reagent 26 applied to the measurement target portion 28, and the blood and the reagent 26 react with each other to exhibit a color. Note that the application position of the reagent 26 and the measurement target portion 28 may be shifted from each other in the longitudinal direction of the cavity 20, and for example, a reaction portion to which the reagent 26 is applied may be disposed on an upstream side of the measurement target portion 28 in the blood flow direction.

In the measurement sensor 12, the pair of plate pieces 30 and the pair of spacers 32 constitute the sensor main body 18 so as to have the above-described cavity 20. The pair of plate pieces 30 are each formed in the above-described rectangular shape in side view, and are arranged in a stacking direction. That is, the pair of plate pieces 30 constitutes both side surfaces (an upper surface and a lower surface) of the sensor main body 18. The plate thickness of each plate piece 30 is very small, and is preferably set to the same dimension of, for example, about 5 to 50 $\mu$m. The thicknesses of the two (one pair of) plate pieces 30 may be different from each other.

The pair of plate pieces 30 maintains a plate shape and has strength not to be plastically deformed even when a certain degree of pressing force is applied thereto from a direction orthogonal to a plane direction. In addition, each plate piece 30 includes a transparent portion or a semitransparent portion such that measurement light can pass through each plate piece 30. Furthermore, each plate piece 30 is preferably formed in a flat plate surface having appropriate hydrophilicity such that blood can flow through the cavity 20.

A material constituting each plate piece 30 is not particularly limited, but a thermoplastic resin material, glass, quartz, or the like is preferably applied. Examples of the thermoplastic resin material include: a polymer material such as a polyolefin (for example, polyethylene or polypropylene), a cycloolefin polymer, a polyester (for example, polyethylene terephthalate (PET) or polyethylene naphthalate), polyvinyl chloride, polystyrene, an ABS resin, an acrylic resin, polyamide, or a fluororesin; and a mixture thereof.

The pair of spacers 32 is disposed so as to be sandwiched between the pair of plate pieces 30, and is firmly bonded to facing surfaces of the plate pieces 30 by a predetermined bonding means (adhesive or the like). That is, each spacer 32 is a member that is disposed between the pair of plate pieces 30 so as to separate the pair of plate pieces

30 from each other, thereby forming the cavity 20 between the pair of plate pieces 30 and the pair of spacers 32 themselves. In this case, one of the spacers 32 is in contact with an upper long side 22a of the sensor main body 18 in Fig. 3, and is disposed so as to extend in the distal end and proximal end directions along the upper long side 22a. The other spacer 32 is in contact with a lower long side 22b of the sensor main body 18 in Fig. 3, and is disposed so as to extend in the distal end and proximal end directions along the lower long side 22b.

A material (substrate) constituting the pair of spacers 32 is not particularly limited, and examples thereof include various thermoplastic elastomers such as a styrene-based elastomer, a polyolefin-based elastomer, a polyurethane-based elastomer, a polyester-based elastomer, a polyamide-based elastomer, a polybutadiene-based elastomer, a transpolyisoprene-based elastomer, a fluororubber-based elastomer, and a chlorinated polyethylene-based elastomer. Alternatively, various elastically deformable materials other than the thermoplastic elastomers may be applied, and a structure such as an elastically deformable porous body (for example, sponge) may be applied. Furthermore, the spacer 32 may include an adhesive on one or both surfaces of the substrate, the adhesive bonding the plate pieces 30 to each other in a cured state or a semi-cured state between the pair of plate pieces 30. Furthermore, the spacer 32 may be configured to elute the reagent 26 into the cavity 20 by containing the reagent 26.

The plate piece 30 and the spacer 32 may be subjected to a hydrophilization treatment. Examples of a method for performing the hydrophilization treatment include a method for applying an aqueous solution containing a hydrophilic polymer such as polyacrylic acid, polyvinylpyrrolidone, or polyacrylamide in addition to a surfactant, polyethylene glycol, polypropylene glycol, hydroxypropyl cellulose, or water-soluble silicone by an immersion method, a spray method, or the like, and a method such as plasma irradiation, glow discharge, corona discharge, or ultraviolet irradiation (for example, excimer light irradiation), and these methods may be used singly or in combination.

Next, a device main body 16 of the blood sugar meter 10 will be described. As illustrated in Fig. 1, the blood sugar meter 10 includes a housing 40 constituting an appearance. The housing 40 includes: a box unit 44 that has a size that allows a user to easily grip and operate the housing 40 and houses a control unit 42 of the blood sugar meter 10 therein; and a tubular photometric unit 46 that protrudes from one side (distal end side) of the box unit 44 in a distal end direction and houses the measurement unit 14 of an optical system therein. In addition, a power button 48, an operation button 50, and a display 52 are disposed on an upper surface of the box unit 44, and an eject lever 54 is disposed on an upper surface of the photometric unit 46.

The power button 48 switches between activation and deactivation of the blood sugar meter 10 under an operation of a user. The operation button 50 functions as an operation unit that measures and displays a blood sugar level, switches display of a measurement result (including a past measurement result), and the like on the basis of the operation of the user in the blood sugar meter 10 in an activated state. The display 52 is constituted by a liquid crystal, an organic EL, or the like, and displays information to be provided to the user in a measurement operation, such as display of a measurement result or error display.

The eject lever 54 is disposed movably in distal end and proximal end directions, and unlocks an eject pin (not illustrated) disposed in the photometric unit 46 to allow the eject pin to advance in the distal end direction.

Meanwhile, the photometric unit 46 of the device main body 16 extends long in the distal end direction from the box unit 44 in order to press a distal end thereof against a finger of the user or the like. As illustrated in Fig. 2, the photometric unit 46 includes a sensor attaching unit 60 having an insertion hole 58 and the measurement unit 14 that optically detects glucose (blood sugar) in blood.

The sensor attaching unit 60 is formed of a material (for example, stainless steel) having high hardness (rigidity), includes a flange portion 60a protruding outward on a distal end side, and is formed in a tubular shape having a predetermined length in an axial direction. The sensor attaching unit 60 is positioned and fixed over a distal end surface and an axial center portion (central portion) of the photometric unit 46 formed of a resin material. As illustrated in Fig. 4A, a fixing wall 46a that firmly fixes the sensor attaching unit 60 is formed so as to protrude on an inner surface of the photometric unit 46.

Examples of a material constituting the sensor attaching unit 60 include materials that are hard, do not easily change in dimensions, are hardly worn even when the measurement sensor is repeatedly inserted and removed, and can be processed with high dimensional accuracy, such as a metal such as stainless steel or titanium, aluminum that has been subjected to an alumite coating treatment, a liquid crystal polymer, a plastic containing a filler such as glass or mica, a plastic whose surface has been cured and coated with nickel plating or the like, a carbon fiber, and a fine ceramic. Among these, if a metal material is applied, the insertion hole 58 can be easily formed with high dimensional accuracy at the time of manufacturing the sensor attaching unit 60 (injection molding, press molding, or the like). Note that, in the device main body 16, the sensor attaching unit 60 may be integrally molded by forming the photometric unit 46 itself of a hard material (for example, a metal material).

The insertion hole 58 is formed in an axial center portion of the sensor attaching unit 60 by being surrounded by a wall portion 62 of the sensor attaching unit 60. The insertion hole 58 is formed so as to have a rectangular cross section that is long in an insertion direction (direction B) and short in a left-right width direction (direction A). The insertion hole 58 has a predetermined depth from a distal end surface thereof toward a depth portion (proximal end direction)

thereof in a state where the sensor attaching unit 60 is fixed to the photometric unit 46.

An insertion opening 58a connected to the insertion hole 58 and communicating with the outside is formed on a distal end side of the sensor attaching unit 60. The dimension of the insertion opening 58a in the insertion direction (direction B) coincides with the dimension (length in direction A) of the short side 24 of the measurement sensor 12. In addition, the dimension of the insertion opening 58a in the left-right width direction, that is, a gap between the pair of wall portions 62 constituting side surfaces of the insertion hole 58 is substantially the same as the thickness of the measurement sensor 12 in a stacking direction (Tall in Fig. 4A) as illustrated in Fig. 4A.

The sensor attaching unit 60 forms a pair of element housing spaces 64 in cooperation with a fixing wall 46a of the photometric unit 46 at an intermediate position where the insertion hole 58 (measurement hole 59) extends. The pair of element housing spaces 64 is a part of the measurement unit 14, is formed at positions facing each other with the insertion hole 58 interposed therebetween, and communicates with the measurement hole 59 via each light guide unit 66 formed by the sensor attaching unit 60.

The measurement unit 14 constitutes a light emitting unit 70 by housing a light emitting element 68 in one of the element housing spaces 64, and constitutes a light receiving unit 74 by housing a light receiving element 72 in the other element housing space 64. The light guide unit 66 of the sensor attaching unit 60 functions as a so-called aperture by being formed in a circular hole having an appropriate diameter.

The light emitting element 68 of the light emitting unit 70 includes a first light emitting element 68a that irradiates the measurement sensor 12 with measurement light having a first wavelength and a second light emitting element 68b that irradiates the measurement sensor 12 with measurement light having a second wavelength different from the first wavelength (not illustrated in Fig. 2). The first light emitting element 68a and the second light emitting element 68b are arranged side by side at positions facing the light guide unit 66 of the element housing space 64.

The light emitting elements 68 (first and second light emitting elements 68a and 68b) may be each constituted by a light emitting diode (LED). The first wavelength is a wavelength for detecting a color-exhibiting concentration of the reagent 26 according to the amount of blood sugar, and is, for example, 600 nm to 680 nm. The second wavelength is a wavelength for detecting a red blood cell concentration in blood, and is, for example, 510 nm to 540 nm. The control unit 42 in the box unit 44 supplies a drive current to cause each of the first and second light emitting elements 68a and 68b to emit light at a predetermined timing. In this case, a blood sugar level obtained from the color-exhibiting concentration is corrected using a hematocrit value obtained from the red blood cell concentration to determine a blood sugar level. Note that noise caused by a blood cell may be corrected by further performing measurement at another measurement wavelength.

The light receiving unit 74 is configured by disposing one light receiving element 72 at a position facing the light guide unit 66 of the element housing space 64. The light receiving unit 74 receives transmission light from the measurement sensor 12, and can be constituted by, for example, a photodiode (PD).

In addition, an eject pin 56 (eject unit) connected to the eject lever 54 is disposed at a bottom (proximal end surface) of the insertion hole 58. The eject pin 56 includes a rod portion 56a extending in an axial direction of the photometric unit 46, and a receiving portion 56b having a large diameter radially outward at a distal end of the rod portion 56a. The proximal end side 24b of the measurement sensor 12 inserted into the insertion hole 58 is in contact with the receiving portion 56b. In addition, a coil spring 76 that surrounds the eject pin 56 in a noncontact manner is disposed between a bottom of the insertion hole 58 and the receiving portion 56b of the eject pin 56. The coil spring 76 elastically supports the receiving portion 56b of the eject pin 56.

When insertion of the measurement sensor 12 is completed, as illustrated in Fig. 4B, the measurement target portion 28 of the measurement sensor 12 is disposed at a position overlapping with the light guide unit 66.

The eject pin 56 is displaced in a proximal end direction by the receiving portion 56b being pressed as a user inserts the measurement sensor 12, and is locked (fixed) by a lock mechanism (not illustrated) disposed in the housing 40. The coil spring 76 elastically contracts according to displacement of the receiving portion 56b. When the eject pin 56 slightly moves by an operation of the eject lever 54 by the user, the lock of the lock mechanism is released, and the eject pin 56 slides in a distal end direction by elastic restoring force of the coil spring 76. As a result, the measurement sensor 12 is pushed out by the eject pin 56 and taken out from the insertion hole 58.

Returning to Fig. 1, the control unit 42 of the device main body 16 is constituted by, for example, a control circuit including a calculation unit, a storage unit, and an input/output unit (not illustrated). A known computer can be applied to this control unit 42. For example, the control unit 42 detects and calculates glucose in blood by driving and controlling the measurement unit 14 under an operation of the operation button 50 by a user, and displays the calculated blood sugar level on the display 52.

For example, in the blood sugar meter 10 that measures an analyte (for example, glucose) by causing the measurement sensor 12 to transmit measurement light, the control unit 42 calculates a measurement result on the basis of the Beer-Lambert law represented by the following formula (A).

[Mathematical Formula 1]

Formula (A)

$$\log_{10}(I_1/I_0) = -\alpha L$$

In the above formula (A), $I_0$ represents an intensity of light before measurement light is incident on a whole blood sample, $I_1$ represents an intensity of light after the measurement light exits from the whole blood sample, $\alpha$ represents an absorption coefficient, and L represents a distance (cell length) through which the measurement light passes.

Examples

The effects of the present invention will be described with reference to the following Examples and Comparative Examples. Note that the technical scope of the present invention is not limited only to the following Examples. Note that, in the following Examples, unless otherwise specified, an operation was performed at room temperature (25°C). In addition, unless otherwise specified, "%" and "parts" mean "% by mass" and "parts by mass", respectively.

Synthesis Example 1: Synthesis of tetrazolium salt 1

A compound (tetrazolium salt 1) having the following structure was synthesized according to the following method.

[Chemical Formula 16]

Tetrazolium salt 1

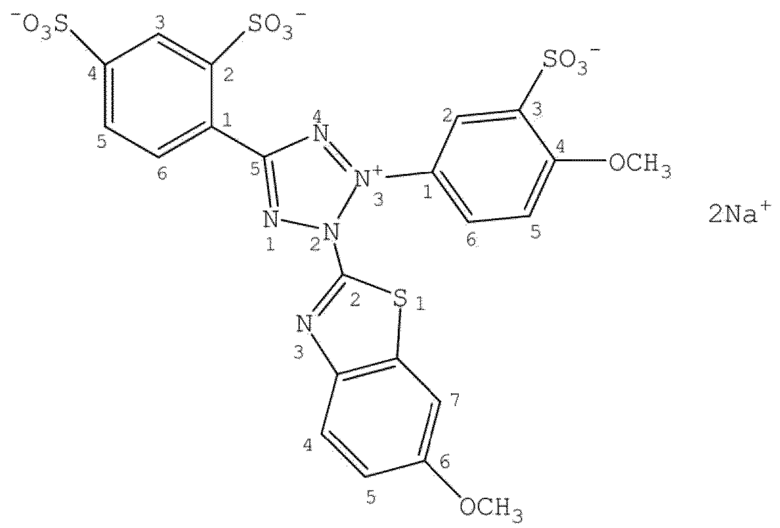

1. Synthesis of hydrazone compound 1

In 60 mL of RO water, 1.59 g of disodium 4-formylbenzene-1,3-disulfonate (manufactured by Tokyo Chemical Industry Co., Ltd.) and 1.0 g of 2-hydrazino-6-methoxy-1,3-benzothiazole (manufactured by Santa Cruz Biotechnology) were suspended. This suspension was heated and stirred in a water bath at 60°C for two hours under an acetic acid acidity. After completion of heating and stirring, a solvent was removed. This residue was washed with isopropanol, and then a precipitate was separated by filtration. This precipitate was dried in a draft to obtain a hydrazone compound 1.

2. Synthesis of formazan compound 1

In a mixed liquid of 10 mL of RO water and 10 mL of DMF, 0.76 g of the hydrazone compound 1 in the above section 1 was dissolved to prepare a hydrazone compound 1 solution. In 4.09 mL of RO water, 0.264 g of p-anisidin-3-sulfonic acid (manufactured by Tokyo Chemical Industry Co., Ltd.) was suspended, and 130 μL of 10 N NaOH was

added thereto and dissolved therein. While this solution was maintained at 0°C, 280 μL of 9.6 N HCl was added thereto, and a sodium nitrite solution was added dropwise thereto to perform diazotization. This diazotized solution was maintained at -20°C, and added dropwise to the hydrazone compound 1 solution. After completion of the dropwise addition, 300 μL of 10 N NaOH was added dropwise thereto, and the mixture was stirred for two hours at room temperature (25°C) to prepare a solution containing a formazan compound 1 (formazan compound 1 solution). The pH of this formazan compound 1 solution was adjusted to neutrality with 9.6 N HCl, and a solvent was removed. The obtained residue was washed with isopropanol, and then a precipitate was separated by filtration. This precipitate was dried to obtain the formazan compound 1.

3. Purification of formazan compound 1 and synthesis of tetrazolium salt 1

The formazan compound 1 in the above section 2 was dissolved in 10 mL of RO water to prepare a formazan compound 1 solution. A column chromatography filler (manufactured by Nacalai Tesque, Inc., COSMOSIL 40C18-PREP) was filled into a disposable column (size: 20 cm × 5 cm), and the disposable column was set in a column preparative separation system (manufactured by Büch Japan Ltd., trade name: SEPACORE). The formazan compound 1 solution was purified using this column system. A solvent of a collected fraction was removed, 15 mL of methanol, 250 μL of 9.6 N HCl, and 5 mL of a 15% ethyl nitrite ($CH_3CH_2NO_2$)-ethanol solution were added to the obtained solid component, and the mixture was stirred for 72 hours under light shielding at room temperature (25°C).

4. Collection of tetrazolium salt 1

Diethyl ether was added to the reaction solution of the above section 3 to precipitate the tetrazolium salt 1. This precipitate was centrifuged, a supernatant was removed, and then the residue was further washed with diethyl ether. The obtained precipitate was dried in a draft to obtain the tetrazolium salt 1.

<Test Example 1: Evaluation of transparency of dry reagent>

1. Preparation of coating liquid

(1) Preparation of coating liquid 1

46.8 μL (corresponding to zinc acetate = 8.6 mg as a transition metal compound) of a 1 M zinc acetate aqueous solution (1 M = 183.48 g/L) and 339.6 μL (339.6 mg) of RO water were mixed to prepare a mixed liquid 1. Next, 3.6 mg of sodium nitrite (1 M = 68.995 g/L) was added to this mixed liquid 1 to prepare a mixed liquid 2. Next, 33.4 mg of sucralose (1 M = 397.64 g/L) was added to this mixed liquid 2 to prepare a mixed liquid 3. Next, 2.5 mg of sorbitol (1 M = 182.17 g/L) as a sugar alcohol was added to this mixed liquid 3 to prepare a mixed liquid 4. Next, 28.1 mg of the tetrazolium salt 1 (1 M = 693 g/L) of Synthesis Example 1 as a color-developing pigment was added to this mixed liquid 4 to prepare a mixed liquid 5.

Separately, 100.2 mg of a nonionic surfactant (polyoxyethylene (9) octylphenyl ether, manufactured by Sigma-Aldrich, Nonidet™ P-40, specific gravity = 1.06 g/mL; hereinafter, also simply referred to as "Nonidet" or "Nonidet P-40") as a hemolytic agent was added to 246.4 μL (246.4 mg) of RO water to prepare a 30% by mass Nonidet aqueous solution.

The mixed liquid 5 prepared above, a 30% by mass Nonidet aqueous solution, and flavin adenine dinucleotide-dependent glucose dehydrogenase (FAD-GDH) (Toyobo Co., Ltd., trade name: GLD-351 (D-Glucose: (flavine adenine dinucleotide)-dehydrogenase)) as an oxidoreductase were mixed so as to have the composition of Table 1-1 below to prepare a coating liquid (total amount = 731.3 μL).

(2) Preparation of coating liquid 2

A coating liquid 2 was prepared in a similar manner to the coating liquid 1 except that the addition amount of sucralose (1 M = 397.64 g/L) was changed to 16.7 mg (57.5 mM) in the composition of the coating liquid 1.

(3) Preparation of coating liquid 3 (Comparative Example)

A coating liquid 3 was prepared in a similar manner to the coating liquid 1 except that sucralose (1 M = 397.64 g/L) was not added in the composition of the coating liquid 1.

[Table 1]

| Table 1-1: Composition of coating liquid | | | | |
|---|---|---|---|---|
| | | Coating liquid | | |
| | | 1 | 2 | 3 |
| Zinc acetate | mg | 8.6 | 8.6 | 8.6 |
| Tetrazolium salt 1 | mg | 28.1 | 28.1 | 28.1 |
| Sodium nitrite | mg | 3.6 | 3.6 | 3.6 |
| Sucralose | mg | 33.4 | 16.7 | 0 |
| Nonidet P-40 | mg | 30.045 | 30.045 | 30.045 |
| FAD-GDH | mg | 8.0 | 8.0 | 8.0 |
| Sorbitol | mg | 2.5 | 2.5 | 2.5 |
| RO water | μL | 702.95 | 702.95 | 702.95 |
| Content mass ratio of sucralose to low-molecular salt compound | | 2.7 | 1.4 | - |
| | | Coating liquid | | |
| | | 1 | 2 | 3 |
| Zinc acetate | mM | 64.0 | 64.0 | 64.0 |
| RO water + Nonidet P-40 | μL | 731.3 | 731.3 | 731.3 |
| Tetrazolium salt 1 | mM | 55.3 | 55.3 | 55.3 |
| Sodium nitrite | mM | 71.3 | 71.3 | 71.3 |
| Sucralose | mM | 114.9 | 57.5 | 0 |
| Nonidet P-40 | w/w | 3.7% | 3.7% | 3.7% |
| FAD-GDH | w/w | 1.0% | 1.0% | 1.0% |
| Sorbitol | mM | 18.8 | 18.8 | 18.8 |
| Molar ratio (%) of sugar alcohol to sucralose | | 16.4 | 32.7 | - |

2. Preparation of dry reagent

(1) Reagent application: 6.5 μL of a coating liquid was applied in a range of width 1.1 mm × length 75 mm onto a polyethylene terephthalate (PET) film (thickness: 188 μm), and the resulting film was stored at room temperature for 12 hours to obtain a dry reagent. A dry reagent to which the coating liquid 1 was applied is referred to as a dry reagent 1, a dry reagent to which the coating liquid 2 was applied is referred to as a dry reagent 2, and a dry reagent to which the coating liquid 3 was applied is referred to as a dry reagent 3.

(2) Test paper assembly:

Test paper was assembled with three members of a first layer: PET film 100 um (without reagent), a second layer: space layer 45 um (double-sided tape), and a third layer: PET film 188 um (dry reagent).

3. Evaluation method 1: measurement of transmittance

Transmittance was measured by transmission spectroscopy (900 nm) using the test paper assembled in the section 2. Each dry reagent was subjected to image observation using a shape measuring machine (KEYENCE CORPORATION, VR-3200). Results thereof are presented in Table 2 below. In addition, results of the image observation are illustrated in Fig. 6A.

4. Evaluation method 2: measurement of contact angle

A contact angle meter (DM301) manufactured by Kyowa Interface Science Co., Ltd. was used as a measurement device. All contact angles were analyzed by a θ/2 method. First, ultrapure water was discharged from a syringe having a distal end attached to the contact angle meter in a downward direction to prepare 1 μL of an ultrapure water droplet at the distal end of the syringe. Next, this syringe was vertically lowered, and the droplet was brought into contact with a reagent application portion of the dry reagent, and 1 μL of ultrapure water was added dropwise to a surface. The droplet that had spread on the surface at this time was photographed from a side, and a contact angle with respect to ultrapure water was obtained using FAMAS which is analysis software attached to the device.

[Table 2]

| | Dry reagent 1 | Dry reagent 2 | Dry reagent 3 |
|---|---|---|---|
| Transmittance (@900 nm) | 73% | 73% | 61% |
| | transparent | transparent | opaque |
| Contact angle (°) | 28.1 | 27.6 | 29.5 |

As described above, the dry reagents 1 and 2 to which the coating liquids 1 and 2 were applied, respectively, had a transmittance of more than 61% and a transparent appearance, whereas the dry reagent 3 to which the coating liquid 3 was applied was opaque. From the above results, it has been found that a dry reagent having high transparency can be obtained by adding sucralose, and the dry reagent can be made amorphous.

Note that a transmittance of a dry reagent having a thickness of 3 to 14 um is preferably 68 to 74%. Note that the thickness of the dry reagent refers to a thickness from a surface of the PET film to a surface of the dry reagent in the vicinity of a center of an application region after the reagent liquid after application is dried, measured using a shape measuring machine (KEYENCE CORPORATION, LS-1100).

<Test Example 2: evaluation of color development reaction>

For the dry reagents 1 to 3 prepared in Test Example 1, a blood sample (plasma 400 mg/dL) was applied to a flow path inlet of the measurement sensor. An absorbance at 605 nm was measured using a fiber spectrophotometer for 15 seconds after the blood sample reached the reagent unit.

Results thereof are illustrated in Fig. 6B. In addition, reagent images after color development are illustrated in Fig. 6A. As illustrated in Fig. 6B, it is found that the dry reagents 1 and 2 containing sucralose each have a high color-developing speed. Therefore, it is found that addition of sucralose accelerates a detection reaction. This is considered to be based on the fact that addition of sucralose can make a dry reagent transparent (amorphous), and a dissolution rate of whole blood in the dry reagent is improved as described above.

<Test Example 3: evaluation of wettability and spreadability of dry reagent>

1. Preparation of coating liquid

Coating liquids 4 to 14 having the compositions described in Table 3 below were prepared in a similar procedure to that of the coating liquid 1.

2. Preparation of dry reagent

Dry reagents 4 to 14 were prepared using the coating liquids 4 to 14 prepared in the section 1, respectively, in a similar manner to Test Example 1.

3. Evaluation method 1: contact angle

A contact angle was measured in a similar manner to Test Example 1. Results thereof are also presented in Table 3 below.

4. Evaluation method 2:

Transmittance was measured in a similar manner to Test Example 1. Results thereof are also presented in Table 3 below. Note that transmittance of a sensor to which a reagent was not applied was 76%.

[Table 3-1]

| | | Coating liquid | | | | | | | | | | |
|---|---|---|---|---|---|---|---|---|---|---|---|---|
| | | 4 | 5 | 6 | 7 | 8 | 9 | 10 | 11 | 12 | 13 | 14 |
| Zinc acetate | mg | 6.9 | 6.9 | 6.9 | 6.9 | 6.9 | 6.9 | 6.9 | 6.9 | 6.9 | 6.9 | 6.9 |
| RO water | μL | 699.3 | 699.3 | 699.3 | 699.3 | 699.3 | 699.3 | 699.3 | 699.3 | 699.3 | 699.3 | 699.3 |
| Tetrazolium salt 1 | mg | 22.4 | 22.4 | 22.4 | 22.4 | 22.4 | 22.4 | 22.4 | 22.4 | 22.4 | 22.4 | 22.4 |
| Sodium nitrite | mg | 2.9 | 2.9 | 2.9 | 2.9 | 2.9 | 2.9 | 2.9 | 2.9 | 2.9 | 2.9 | 2.9 |
| Sucralose | mg | 33.4 | 33.4 | 33.4 | 33.4 | 33.4 | 33.4 | 33.4 | 33.4 | 33.4 | 33.4 | 33.4 |
| Nonidet P-40 | mg | 33.7 | 33.7 | 33.7 | 33.7 | 33.7 | 33.7 | 33.7 | 33.7 | 33.7 | 33.7 | 33.7 |
| FAD-GDH | mg | 8 | 8 | 8 | 8 | 8 | 8 | 8 | 8 | 8 | 8 | 8 |
| Erythritol | mg | 15 | | 2.5 | | | 5.0 | | | | | |
| Xylitol | mg | | | | | | | 5.0 | | | | |
| Sorbitol | mg | | 15 | | 2.5 | | | | | 5.0 | 3.8 | |
| Mannitol | mg | | | | | 2.5 | | | 5.0 | | | |
| Content mass ratio of sucralose to low-molecular salt compound | | 3.4 | 3.4 | 3.4 | 3.4 | 3.4 | 3.4 | 3.4 | 3.4 | 3.4 | 3.4 | 3.4 |

[Table 3-2]

| | | Coating liquid | | | | | | | | | | |
|---|---|---|---|---|---|---|---|---|---|---|---|---|
| | | 4 | 5 | 6 | 7 | 8 | 9 | 10 | 11 | 12 | 13 | 14 |
| Zinc acetate | mM | 51.2 | 51.2 | 51.2 | 51.2 | 51.2 | 51.2 | 51.2 | 51.2 | 51.2 | 51.2 | 51.2 |
| RO water + Nonidet P-40 | μL | 731.09 | 731.09 | 731.09 | 731.09 | 731.09 | 731.09 | 731.09 | 731.09 | 731.09 | 731.09 | 731.09 |
| Tetrazolium salt 1 | mM | 44.3 | 44.3 | 44.3 | 44.3 | 44.3 | 44.3 | 44.3 | 44.3 | 44.3 | 44.3 | 44.3 |
| Sodium nitrite | mM | 57.1 | 57.1 | 57.1 | 57.1 | 57.1 | 57.1 | 57.1 | 57.1 | 57.1 | 57.1 | 57.1 |
| Sucralose | mM | 115.0 | 115.0 | 115.0 | 115.0 | 115.0 | 115.0 | 115.0 | 115.0 | 115.0 | 115.0 | 115.0 |
| Nonidet P-40 | w/w | 4.2% | 4.2% | 4.2% | 4.2% | 4.2% | 4.2% | 4.2% | 4.2% | 4.2% | 4.2% | 4.2% |
| FAD-GDH | w/w | 1.0% | 1.0% | 1.0% | 1.0% | 1.0% | 1.0% | 1.0% | 1.0% | 1.0% | 1.0% | 1.0% |
| Erythritol | mM | 168.0 | | 28.0 | | | 56.0 | | | | | |
| Xylitol | mM | | | | | | | 44.9 | | | | |
| Sorbitol | mM | | 112.6 | | 18.8 | | | | | 37.5 | 28.2 | |
| Mannitol | mM | | | | | 18.8 | | | 37.5 | | | |
| Molar ratio (%) of sugar alcohol to sucralose | | 146.1 | 97.9 | 24.3 | 16.3 | 16.3 | 48.7 | 39.0 | 32.6 | 32.6 | 24.5 | - |

[Table 3-3]

| | Dry reagent | | | | | | | | | | |
|---|---|---|---|---|---|---|---|---|---|---|---|
| | 4 | 5 | 6 | 7 | 8 | 9 | 10 | 11 | 12 | 13 | 14 |
| Contact angle (°) | 26.8 | 25.2 | 28.3 | 27.4 | 26.6 | 23.5 | 24.3 | 27.0 | 23.6 | 27.1 | 37.6 |
| Transmittance (@900 nm) | 69% | 70% | 73% | 74% | 73% | 72% | 74% | 71% | 73% | 73% | 74% |

The contact angles of the dry reagents 4 to 13 were lower than the contact angle of the dry reagent 14, and it had been found that the contact angle was less than 30° by the addition of a sugar alcohol. In the dry reagent, a low contact angle with respect to water means high wettability to blood. Therefore, the addition of a sugar alcohol improves blood spreadability on the dry reagent to further accelerate a detection reaction of the test.

Note that the contact angle with respect to water is preferably less than 40°, and more preferably 23 to 29°.

<Test Example 4: evaluation of false color development>

In the detection method using a color-developing pigment, the less false color development is, the better from a viewpoint of improving detection accuracy. Therefore, false color development when sucralose and a sugar alcohol were added was evaluated.

1. Preparation of test liquid

Test liquids having the compositions presented in Table 4-1 below were prepared.

[Table 4-1]

| | | Test liquid | | | | | | | | |
|---|---|---|---|---|---|---|---|---|---|---|
| | | 15 | 16 | 17 | 18 | 19 | 20 | 21 | 22 | 23 |
| RO water | μL | 733 | 733 | 733 | 733 | 733 | 733 | 733 | 733 | 733 |
| Tetrazolium salt 1 | mg | 22.4 | 22.4 | 22.4 | 22.4 | 22.4 | 22.4 | 22.4 | 22.4 | 22.4 |
| Zinc acetate | mg | 12.0 | 12.0 | 12.0 | 12.0 | 12.0 | 12.0 | 12.0 | 12.0 | 12.0 |
| Erythritol | mg | | 20.0 | | | | | | | |
| Xylitol | mg | | | 20.0 | | | | | | |
| Sorbitol | mg | | | | 20.0 | | | | | |
| Mannitol | mg | | | | | 20.0 | | | | |
| Sucralose | mg | | | | | | 20.0 | | | |
| Disodium succinate | mg | | | | | | | 20.0 | | |
| Trehalose | mg | | | | | | | | 20.0 | |
| Sucrose | mg | | | | | | | | | 20.0 |
| | | 15 | 16 | 17 | 18 | 19 | 20 | 21 | 22 | 23 |
| RO water | μL | 733.0 | 733.0 | 733.0 | 733.0 | 733.0 | 733.0 | 733.0 | 733.0 | 733.0 |
| Tetrazolium salt 1 | mM | 44.1 | 44.1 | 44.1 | 44.1 | 44.1 | 44.1 | 44.1 | 44.1 | 44.1 |
| Zinc acetate | mM | 89.5 | 89.5 | 89.5 | 89.5 | 89.5 | 89.5 | 89.5 | 89.5 | 89.5 |
| Erythritol | mM | | 223.4 | | | | | | | |
| Xylitol | mM | | | 179.3 | | | | | | |
| Sorbitol | mM | | | | 149.8 | | | | | |
| Mannitol | mM | | | | | 149.8 | | | | |
| Sucralose | mM | | | | | | 68.6 | | | |

(continued)

| | | 15 | 16 | 17 | 18 | 19 | 20 | 21 | 22 | 23 |
|---|---|---|---|---|---|---|---|---|---|---|
| Disodium succinate | mM | | | | | | | 101.0 | | |
| Trehalose | mM | | | | | | | | 72.1 | |
| Sucrose | mM | | | | | | | | | 79.7 |

2. Evaluation method (heat resistance: false color development)

The above test liquid was stored in an oven at 60°C. Color development was visually confirmed after three hours and after 60 hours.

Results thereof are presented in Table 4-2 below. A result in which false color development has occurred is described as "present", and a result in which false color development has not occurred is described as "absent".

[Table 4-2]

| | Test liquid | | | | | | | | |
|---|---|---|---|---|---|---|---|---|---|
| | 15 | 16 | 17 | 18 | 19 | 20 | 21 | 22 | 23 |
| False color development after 3 hours | absent | absent | absent | absent | absent | absent | absent | absent | absent |
| False color development after 60 hours | absent | - | - | - | - | absent | absent | absent | present |
| -: No data | | | | | | | | | |

From the above results, in the test liquids 16 to 20 to which a sugar alcohol was added, the test liquid 22 to which trehalose was added, and the test liquid 23 to which sucrose was added, false color development did not occur after three hours. Note that, in the test liquid 21 to which disodium succinate that is not a sugar alcohol was added, white precipitation was generated immediately after manufacture.

<Test Example 5: evaluation of blood spreadability and deterioration>

1. Preparation of coating liquid and dry reagent

Coating liquids 24 to 31 having the following compositions were prepared in a similar manner to the preparation of the coating liquid 1.

2. Evaluation method-1 (evaluation of blood spreadability)

"Blood spreadability" means that, in a blood sugar level measuring sensor, blood flowing from a first end side of a reagent layer toward a second end side thereof flows into a space present above a reagent, and the blood and the blood sugar level measuring reagent are uniformly mixed and dissolved. Furthermore, "good spreadability of blood flowing into a blood sugar level measuring sensor" means that when blood flowing from a first end side toward a second end side through a flow path and the blood sugar level measuring reagent are mixed and dissolved, a flow state of the blood is not disturbed, and the blood flows to the second end while being dissolved with the blood sugar level measuring reagent at a speed within a predetermined range. "The flow state of the blood is not disturbed" includes that a distal end of the inflowing blood uniformly advances in the flow path in a flow path cross section.

(1) Preparation of blood sugar level measuring sensor

A blood sugar meter sensor (blood sugar level measuring sensor) illustrated in Fig. 5 was prepared as follows. First, 3.15 μL of each of the prepared coating liquids 1, 7, 14, and 24 to 33 was applied onto a polyethylene terephthalate (PET) film (manufacturer name: Toray Industries, Inc., trade name: Lumirror T60, thickness: 188 μm, 8 mm × 80 mm)

placed on a stage (on a "reagent application surface 3" side in Fig. 5A) with a head having a patterning accuracy within ± 5 to 8 um and a discharge liquid amount of 1 pL to 1000 pL using an inkjet (manufactured by MicroJet, Inc., Labojet-500Bio), and dried at 25°C for 10 minutes to form a reagent layer on a PET film (PET with a reagent layer). This PET with a reagent layer was cut into a predetermined size (8 mm × 1.6 mm) to prepare a film piece ("film piece 2" in Fig. 5A). This film piece had, per piece, 0.063 μL of the coating liquid applied. A reagent mass per blood sugar level measuring sensor (0.063 μL as a coating liquid) can be calculated from the composition (mass) of each reagent component added at the time of preparing the coating liquid, the final volume (731.3 μL) of a prepared reagent liquid, and the number of film pieces 2 prepared from PET with a reagent layer. For example, the film piece to which the coating liquid 1 has been applied contains, per piece, 2.4 μg of tetrazolium salt 1 (color-developing pigment), 0.7 μg of zinc acetate (transition metal compound), 0.3 μg of sodium nitrite, 2.9 μg of sucralose, 0.2 μg of sorbitol (sugar alcohol), 0.7 μg of FAD-GDH (oxidoreductase), and 2.6 μg of Nonidet (hemolytic agent) in a form of a dry reagent.

Double-sided tapes (manufacturer name: Nitto Denko, trade name: 5605BN, thickness: 50 μm) ("double-sided tapes 4" in Fig. 5A) were disposed as a spacer and an adhesive portion (sensor base) on both sides of a hydrophilicized polyester film (manufactured by 3M, trade name: hydrophilicized polyester film 9901P, thickness: 100 μm) ("polyester film 5" in Fig. 5A) as a second substrate. The film piece ("film piece 2" in Fig. 5A) prepared above was stuck to this sensor base such that the reagent layer ("reagent application surface 3" in Fig. 5A) faced the sensor base and was located at a center of the flow path ("flow path 6" in Fig. 5A). When the film piece was stuck, the film piece was pressed such that a predetermined amount of the film piece was embedded in the double-sided tapes. Furthermore, the sensor base to which the film piece was stuck was covered with a film piece ("polyester film 7" in Fig. 5A) in which a double-sided tape (manufacturer name: 3M, trade name: polyester film substrate, double-sided adhesive tape 9965, thickness: 80 μm) was stuck to a hydrophilicized polyester film to prepare a blood sugar meter sensor (blood sugar level measuring sensor).

The prepared blood sugar meter sensor (blood sugar level measuring sensor) includes a flow path unit and a measurement unit (reagent unit). In the flow path unit in Fig. 5B, a flow path length ("L1" in Fig. 5B) was 9 mm, a width ("W" in Fig. 5B) was 1.1 mm, and a thickness ("t1" in Fig. 5B) was 0.13 mm. In the measurement unit in Fig. 5B, a reagent length ("L2" in Fig. 5B) was 1.6 mm, a width ("W" in Fig. 5B) was 1.1 mm, and a thickness ("t2" in Fig. 5B) was 0.05 mm.

Note that the length L1 of the flow path unit in a direction (longitudinal direction of the flow path) orthogonal to a sensor thickness direction is not particularly limited, and can be appropriately selected according to a purpose, but is preferably 5 to 10 mm. Here, the long length L1 is advantageous in that attachment (insertion) to a component measurement device is facilitated and entry of ambient light into an optical measurement unit is reduced. The short length L1 is advantageous in that a specimen amount can be reduced. Therefore, an upper limit and a lower limit of the length L1 are determined in consideration of a balance among ease of attachment (insertion) to the component measurement device, an influence of ambient light, and a specimen amount. A length L2 of a flow path in the reagent unit in a direction (longitudinal direction of the flow path) orthogonal to a sensor thickness direction is not particularly limited, and can be appropriately selected according to a purpose, but is preferably 1 to 4 mm. Here, the long length L2 is advantageous in that measurement can be performed with high accuracy because an irradiation spot area can be large in the length direction. However, the short length L2 is advantageous in that a specimen amount can be reduced. Therefore, an upper limit and a lower limit of the length L2 are determined in consideration of a balance between measurement accuracy and a specimen amount.

(2) Evaluation of blood spreadability

To the prepared blood sugar level measuring sensor, 1 $mm^3$ of prepared blood (whole blood, hematocrit value (Ht) 60) was spotted, and blood spreadability was visually evaluated according to the following evaluation criteria. Note that an environmental temperature was 25°C.

«Evaluation criteria>>

⊙: A blood specimen immediately spread over the entire area.
◦: A blood specimen quickly spread over a central measurement range, but it took some time for the blood specimen to spread over the entire area.
△: A blood sample quickly spread over a central measurement range, but it took time for the blood specimen to spread over the entire area.

3. Evaluation method-2 (deterioration test)

The prepared blood sugar level measuring sensor was housed in an aluminum packaging and then stored in a thermo-hygrostat (manufactured by Yamato Scientific Co., Ltd.) at 60°C ± 1°C. After a lapse of three days, the blood sugar level measuring sensor was taken out from the thermo-hygrostat, and false color development and coloring of a dry reagent were evaluated. The evaluation was made by measuring an absorbance at a wavelength of 605 nm and using an absorbance increase with respect to the blood sugar level measuring sensor (blank value) stored at 25°C for three days.

«Evaluation criteria>>

⊙: An absorbance after storage at 60°C is less than 0.03 with respect to the blank value.
◦: An absorbance after storage at 60°C is 0.03 or more and less than 0.07 with respect to the blank value.
△: An absorbance after storage at 60°C is 0.07 or more and less than 0.15 with respect to the blank value.
×: An absorbance after storage at 60°C is more than 0.15 with respect to the blank value.

[Table 5-1]

| | | Coating liquid 14 | Coating liquid 7 | Coating liquid 24 | Coating liquid 25 | Coating liquid 26 | Coating liquid 27 | Coating liquid 1 | Coating liquid 28 | Coating liquid 29 | Coating liquid 30 | Coating liquid 31 |
|---|---|---|---|---|---|---|---|---|---|---|---|---|
| Zinc acetate | mg | 6.9 | 6.9 | 6.9 | 8.6 | 13.7 | 6.9 | 8.6 | 8.6 | 8.6 | 8.6 | 8.6 |
| Tetrazolium salt 1 | mg | 22.4 | 22.4 | 22.4 | 28.1 | 22.4 | 22.4 | 28.1 | 28.1 | 28.1 | 28.1 | 22.4 |
| Sodium nitrite | mg | 2.9 | 2.9 | 2.9 | 3.6 | 2.9 | 2.9 | 3.6 | 3.6 | 3.6 | 2.9 | 2.9 |
| Sucralose | mg | 33.4 | 33.4 | 33.4 | 52.2 | 33.4 | 33.4 | 33.4 | 33.4 | 44.6 | 33.4 | 33.4 |
| FAD-GDH | mg | 8.0 | 8.0 | 8.0 | 8.0 | 8.0 | 8.0 | 8.0 | 8.0 | 8.0 | 8 | 8 |
| Sorbitol | mg | 0.0 | 2.5 | 7.5 (2.5 mg of erythritol, 2.5 mg of xylitol, and 2.5 mg of sorbitol) | 0.0 | 0.0 | 0.0 | 2.5 | 2.5 | 2.5 | 2.5 | 2.5 |
| Nonidet P-40 | mg | 33.7 | 33.7 | 33.7 | 42.1 | 33.7 | 27.1 | 30.0 | 33.7 | 33.7 | 33.7 | 30.0 |
| RO water | mg | 699.3 | 699.3 | 699.3 | 690.9 | 699.3 | 705.9 | 703.0 | 699.3 | 699.3 | 699.3 | 703.0 |

[Table 5-2]

| | | Coating liquid 14 | Coating liquid 7 | Coating liquid 24 | Coating liquid 25 | Coating liquid 26 | Coating liquid 27 | Coating liquid 1 | Coating liquid 28 | Coating liquid 29 | Coating liquid 30 | Coating liquid 31 |
|---|---|---|---|---|---|---|---|---|---|---|---|---|
| Zinc acetate | mM | 51.2 | 51.2 | 51.2 | 64.1 | 102.4 | 51.2 | 64.0 | 64.0 | 64.0 | 64.0 | 64.0 |
| Tetrazolium salt 1 | mM | 44.3 | 44.3 | 44.3 | 55.4 | 44.3 | 44.3 | 55.3 | 55.4 | 55.4 | 55.4 | 44.3 |
| Sodium nitrite | mM | 57.1 | 57.1 | 57.1 | 71.4 | 57.1 | 57.1 | 71.3 | 71.4 | 71.4 | 57.1 | 57.1 |
| Sucralose | mM | 115.0 | 133.6 | 115.0 | 179.8 | 115.0 | 114.9 | 114.9 | 115.0 | 153.3 | 115.0 | 114.9 |
| Sorbitol | mM | 0.0 | 18.8 | 69.2 (total of three types of alcohols) | 0.0 | 0.0 | 0.0 | 18.8 | 18.8 | 18.8 | 18.8 | 18.8 |
| Spreadability | | △ | ⊙ | ⊙ | △ | △ | △ | ⊙ | ○ | △ | ○ | ○ |
| 60°C deterioration | | ⊙ | ○ | △ | ⊙ | ⊙ | ⊙ | ⊙ | ⊙ | ⊙ | ⊙ | ⊙ |
| Ratio of sugar alcohol to sucralose | | 0% | 14% | 60% | 0% | 0% | 0% | 16% | 16% | 12% | 16% | 16% |

Comparison between the dry reagent 14 using the coating liquid 14 and the dry reagent 7 using the coating liquid 7 indicates that addition of a sugar alcohol improves blood spreadability. This is considered to be because wettability of whole blood is improved as confirmed above. In addition, comparison between the coating liquids 28 and 29 indicates that the dry reagent 28 using the coating liquid 28 in which a sugar alcohol ratio to sucralose is 14% or more has better blood spreadability than the dry reagent 29 using the coating liquid 29.

<Test Example 6: evaluation by blood sugar meter sensor>

A high concentration glucose solution (40 g/dL) was added to blood (whole blood, hematocrit value (Ht) 40) to prepare a blood specimen (BG100) having a glucose concentration (BG) of 100 mg/dL, a blood specimen (BG200) having a glucose concentration (BG) of 200 mg/dL, a blood specimen (BG400) having a glucose concentration (BG) of 400 mg/dL, and a blood specimen (BG550) having a glucose concentration (BG) of 550 mg/dL. In addition, as a control, a glucose degrading enzyme was added to blood (whole blood, hematocrit value (Ht) 40) to prepare a blood specimen (BG0) having a glucose concentration (BG) of 0 mg/dL.

Blood adjusted to each of BG0, BG100, BG200, BG400, and BG550 was spotted to a flow path inlet of the blood sugar level measuring sensor (the coating liquid 1 was used as the coating liquid) prepared in the above Test Example 5. Nine seconds after each specimen was spotted to the reagent unit, an absorbance at 605 nm was measured using a fiber spectrophotometer. Results thereof are illustrated in Fig. 7. Fig. 7 is a graph illustrating a relationship between a glucose concentration and an absorbance of a chelate compound of formazan and $Ni^{2+}$ at a maximum absorption wavelength (605 nm) when each sample is measured by the blood sugar level measuring sensor.

As illustrated in Fig. 7, the absorbance and the glucose concentration have a linear relationship (proportional relationship). From this, it is considered that a blood sugar level can be accurately measured on the basis of the absorbance by using the sensor of the present disclosure.

From these, a concentration of a biological component in a whole blood sample can be accurately calculated by measuring a color development amount by applying a hemolysis sample of whole blood to the reagent (blood sugar level measuring sensor) of the present disclosure and preparing a calibration curve on the basis of the color development amount.

This application is based on Japanese Application No. 2021-201646 filed on December 13, 2021, the entire content of which is incorporated herein by reference.

Reference Signs List

| | |
|---|---|
| 1 | Reagent ribbon |
| 2 | Film piece |
| 3 | Reagent application surface |
| 4 | Double-sided tape |
| 5 | Polyester film |
| 6 | Flow path |
| 7 | Polyester film |
| 10 | Blood sugar meter |
| 12 | Measurement sensor |
| 14 | Measurement unit |
| 16 | Device main body |
| 18 | Sensor main body |
| 20 | Cavity |
| 20a | Distal end port |
| 20b | Proximal end port |
| 22 | Long side |
| 22a | Upper long side |
| 22b | Lower long side |
| 24 | Short side |
| 24a | Distal end side |
| 24b | Proximal end side |
| 26 | Reagent |
| 28 | Measurement target portion |
| 30 | Plate piece |

| | |
|---|---|
| 32 | Spacer |
| 40 | Housing |
| 42 | Control unit |
| 44 | Box unit |
| 46 | Photometric unit |
| 48 | Power button |
| 50 | Operation button |
| 52 | Display |
| 54 | Eject lever |
| 56 | Eject pin |
| 56a | Rod portion |
| 56b | Receiving portion |
| 58 | Insertion hole |
| 58a | Insertion opening |
| 59 | Measurement hole |
| 60 | Sensor attaching unit |
| 60a | Flange portion |
| 62 | Wall portion |
| 64 | Element housing space |
| 66 | Light guide unit |
| 68 | Light emitting element |
| 70 | Light emitting unit |
| 72 | Light emitting element |
| 74 | Light receiving unit |
| 76 | Coil spring |

## Claims

1. A biological component concentration measuring reagent comprising a color-developing pigment, an oxidoreductase, a hemolytic agent, and a low-molecular salt compound, and further comprising
sucralose.

2. The biological component concentration measuring reagent according to claim 1, wherein the salt compound is at least one selected from the group consisting of a transition metal salt and an inorganic salt.

3. The biological component concentration measuring reagent according to claim 2, wherein the inorganic salt is a nitrite.

4. The biological component concentration measuring reagent according to claim 1 or 2, wherein a content mass ratio of the sucralose to the low-molecular salt compound is 1 to 10.

5. The biological component concentration measuring reagent according to claim 1 or 2, further comprising a sugar alcohol.

6. The biological component concentration measuring reagent according to claim 5, wherein the sugar alcohol is at least one selected from the group consisting of lactitol, erythritol, sorbitol, xylitol, and mannitol.

7. The biological component concentration measuring reagent according to claim 5, wherein a molar ratio of the sugar alcohol to the sucralose is 10% or more.

8. A biological component concentration measuring method comprising: bringing a whole blood sample into contact with the biological component concentration measuring reagent according to claim 1 or 2; measuring a color development amount; and quantifying a concentration of a biological component in the whole blood sample on a basis of the color development amount.

9. The method according to claim 8, wherein the biological component is glucose, cholesterol, neutral lipid, nicotinamide adenine dinucleotide phosphate (NADPH), nicotinamide adenine dinucleotide (NADH), or uric acid.

**10.** A sensor for measuring a biological component concentration in a whole blood sample, the sensor having a reaction portion, wherein
the reaction portion includes the biological component concentration measuring reagent according to claim 1 or 2.

FIG. 1

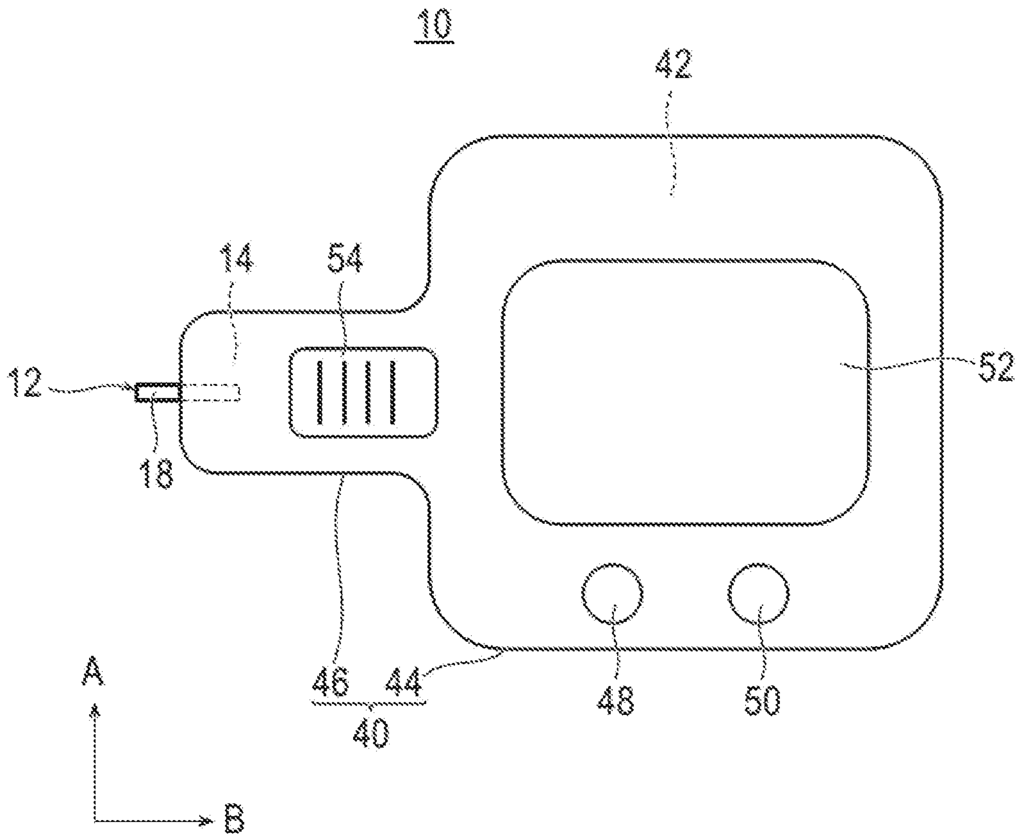

FIG. 2

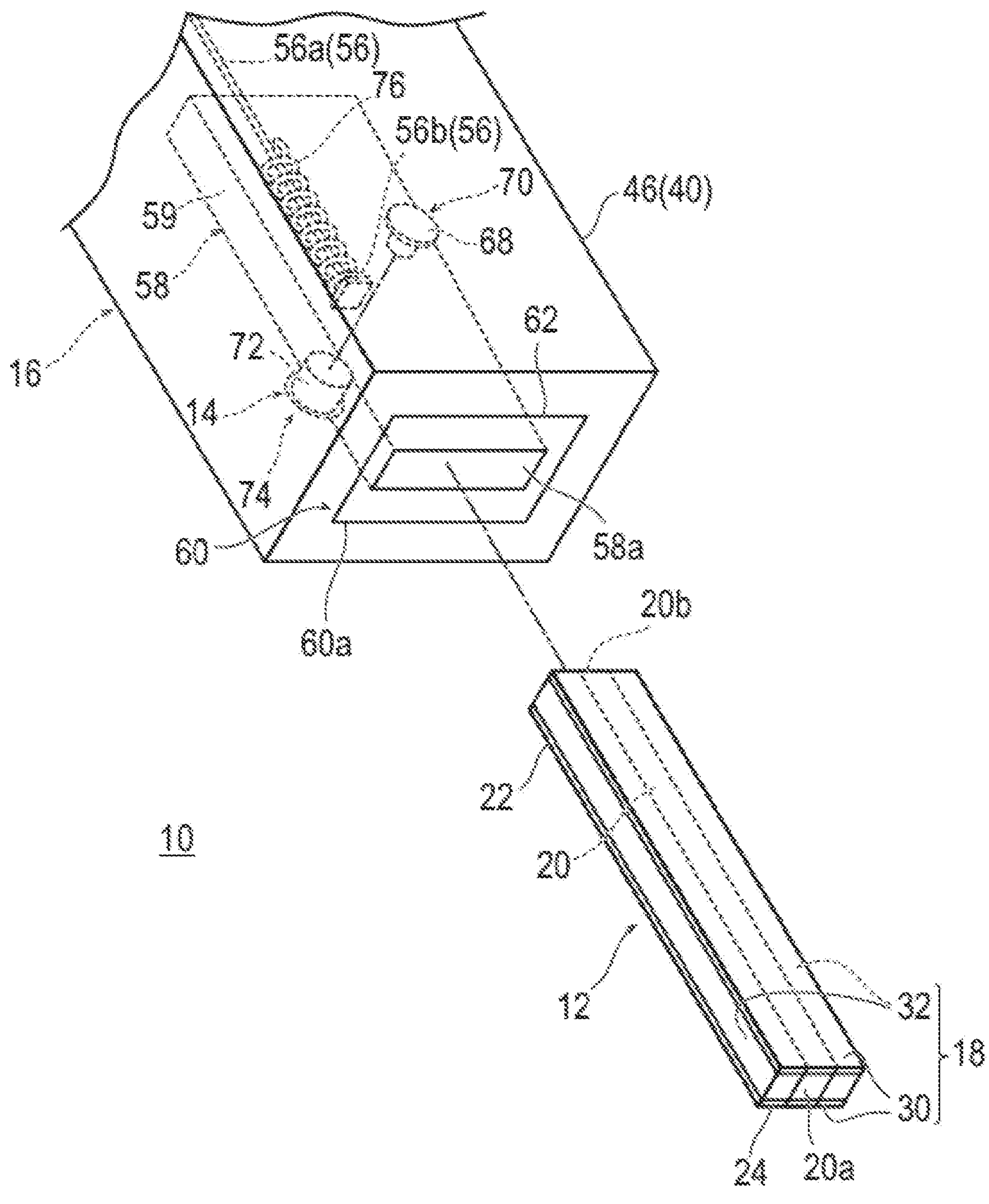
56a(56)
76
56b(56)
70
46(40)
59
68
58
62
16
72
14
74
58a
60
60a
20b
22
10
20
12
32
18
30
24
20a

FIG. 3

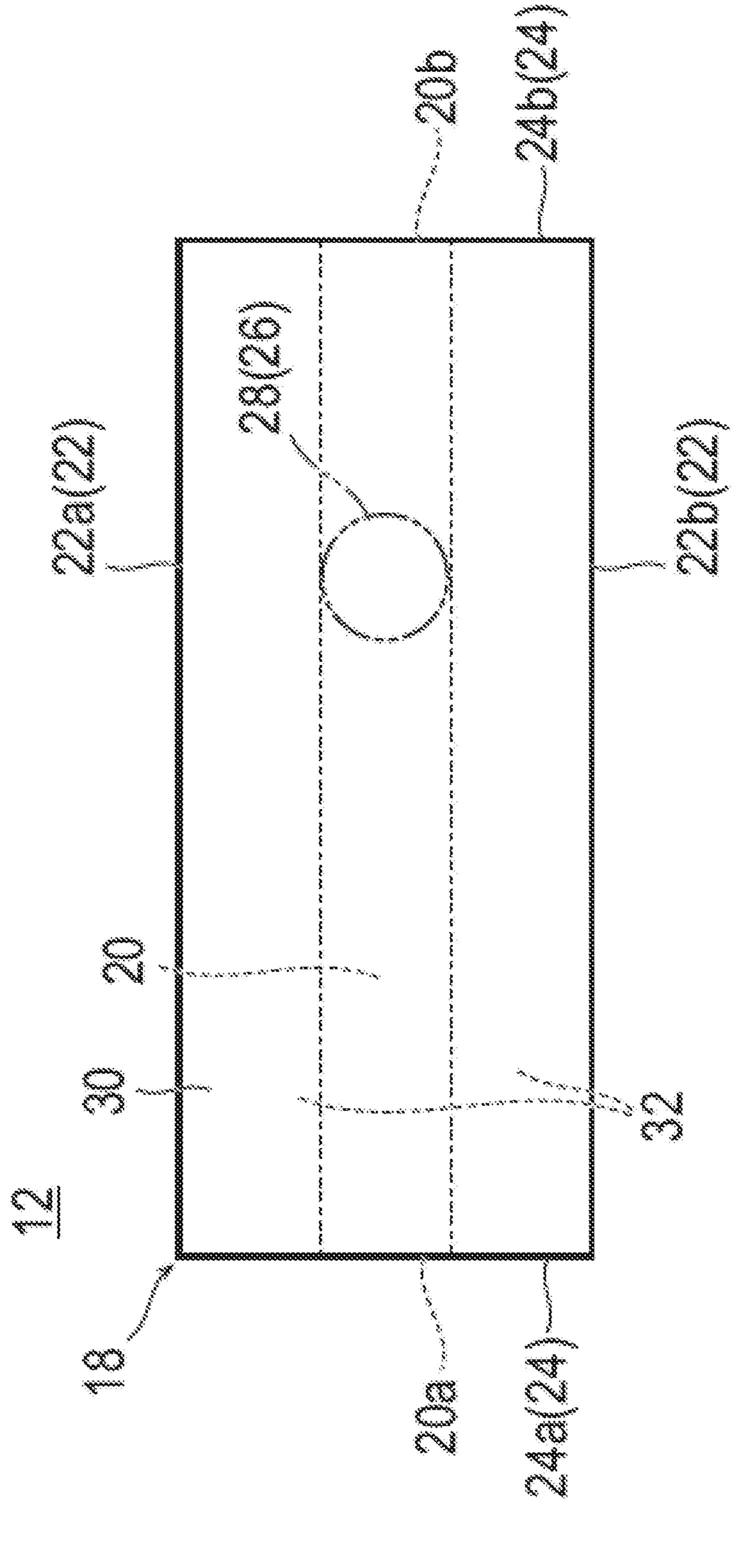

FIG. 4A

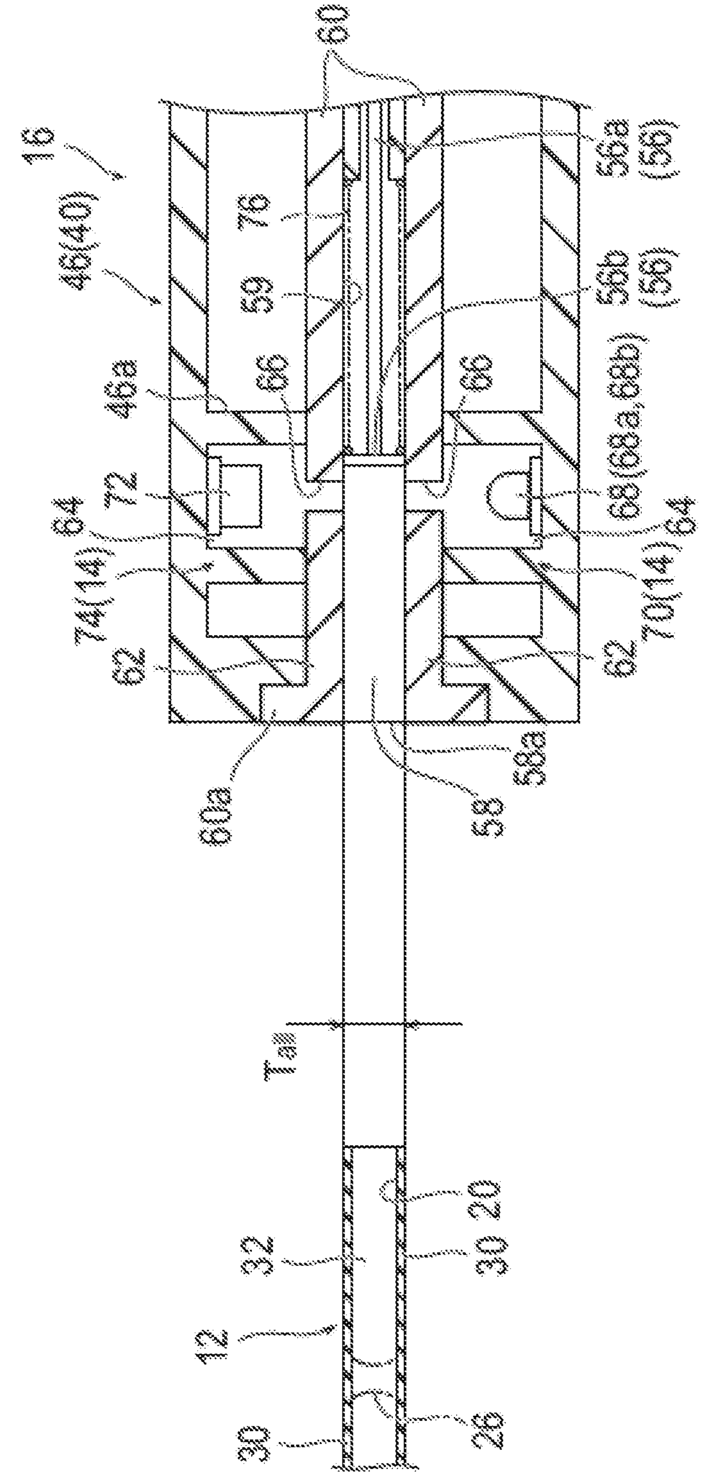
16
46(40)
46a
72
64
74(14)
62
60a
58
58a
62
70(14)
64
68(68a, 68b)
66
66
59
76
56b
(56)
56a
(56)
60
Tall
12
32
30
20
30
26

FIG. 4B

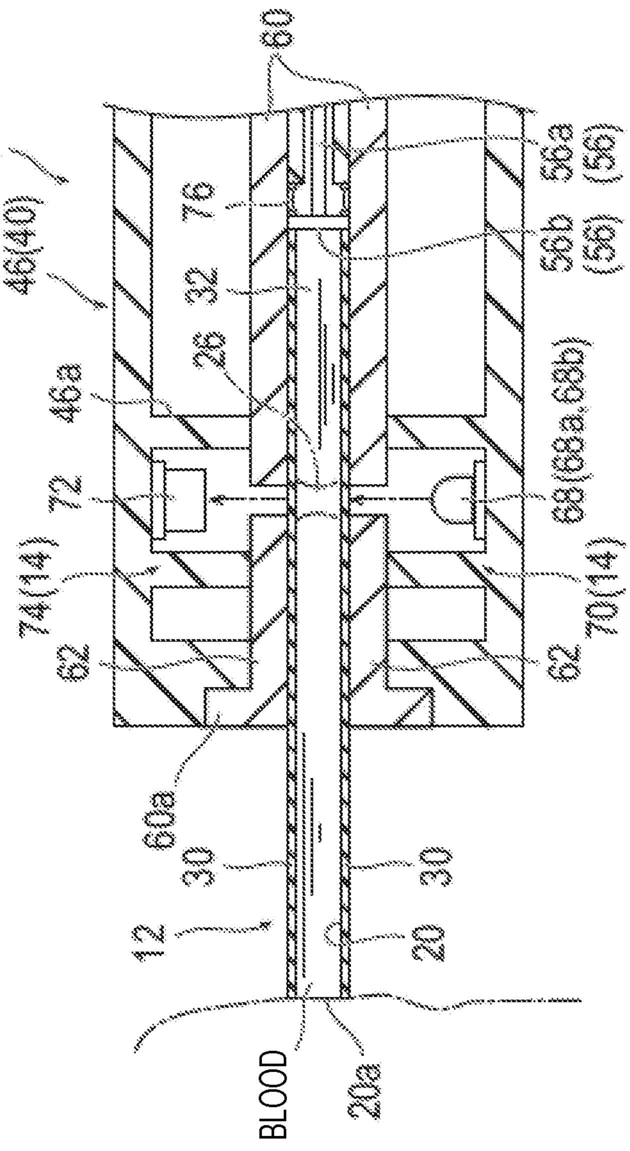
16
46(40)
76
32
26
46a
72
74(14)
62
60a
30
12
BLOOD
20a
20
30
62
70(14)
68(68a,68b)
56b
(56)
56a
(56)
60

FIG. 5A

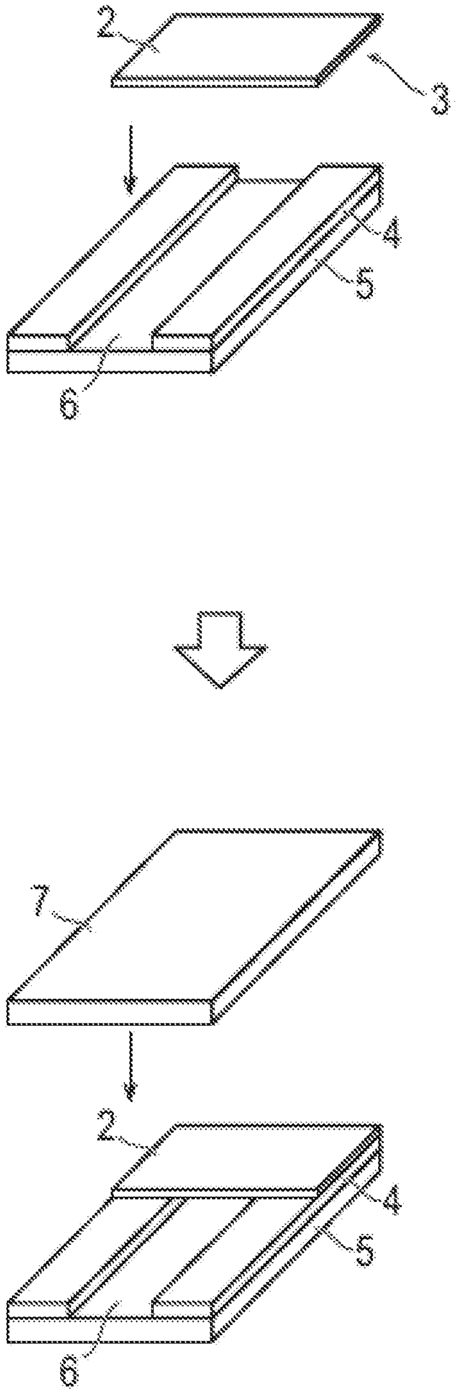

FIG. 5B

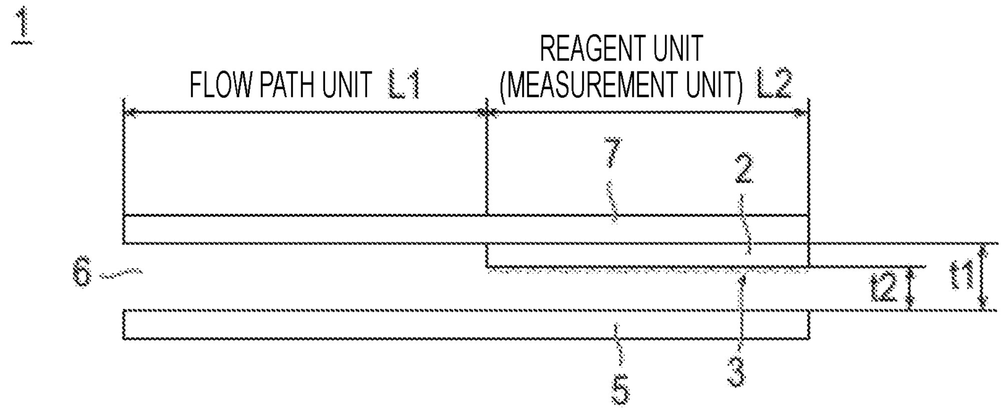
1
REAGENT UNIT
FLOW PATH UNIT L1
(MEASUREMENT UNIT) L2
7
2
6
t1
t2
3
5

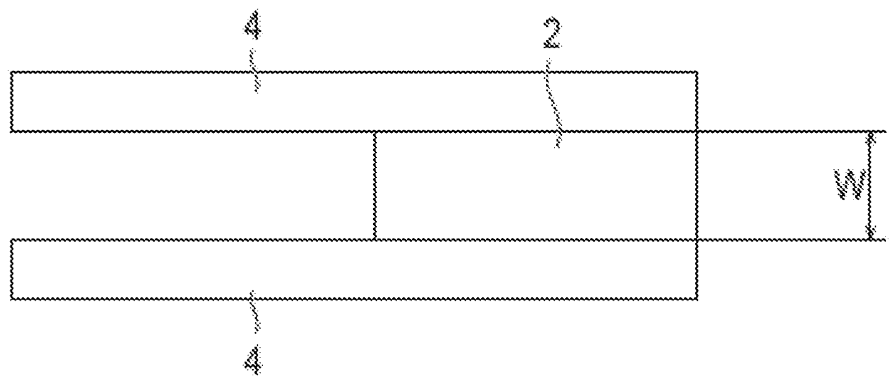
4
2
W
4

FIG. 6A

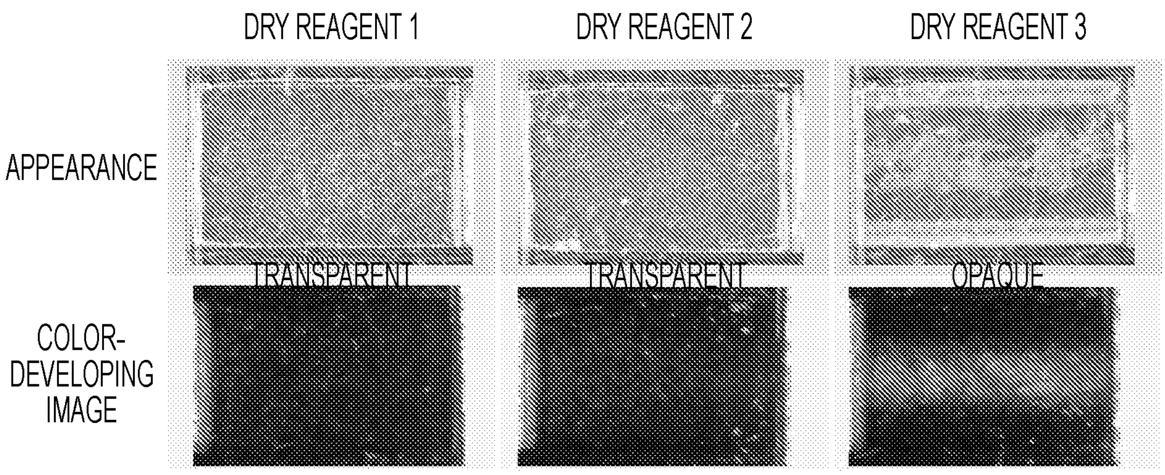
DRY REAGENT 1
DRY REAGENT 2
DRY REAGENT 3
APPEARANCE
TRANSPARENT
TRANSPARENT
OPAQUE
COLOR-
DEVELOPING
IMAGE

*FIG. 6B*

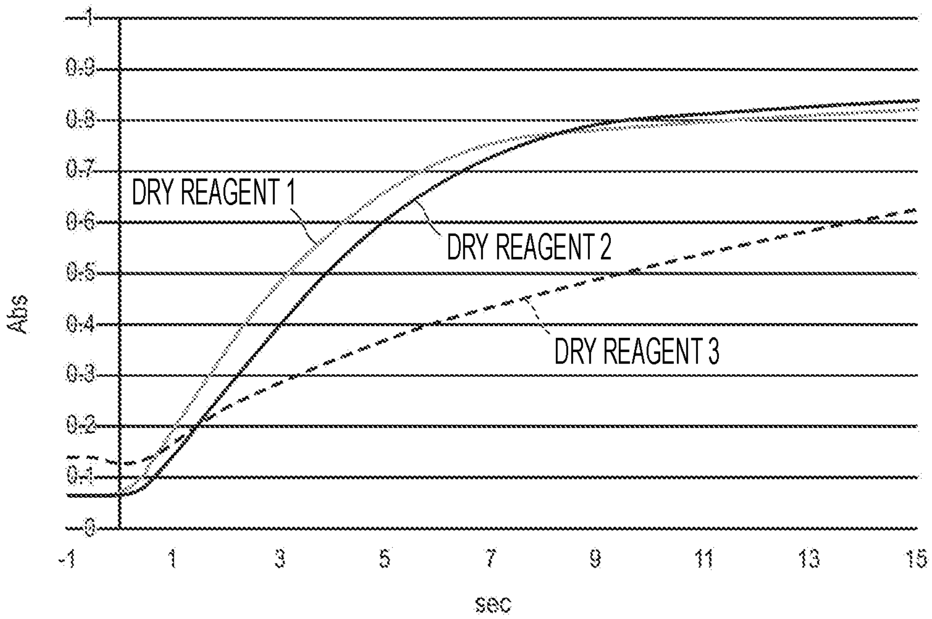
Abs
1
0.9
0.8
0.7
0.6
0.5
0.4
0.3
0.2
0.1
0
-1
1
3
5
7
9
11
13
15
sec
DRY REAGENT 1
DRY REAGENT 2
DRY REAGENT 3

*FIG. 7*

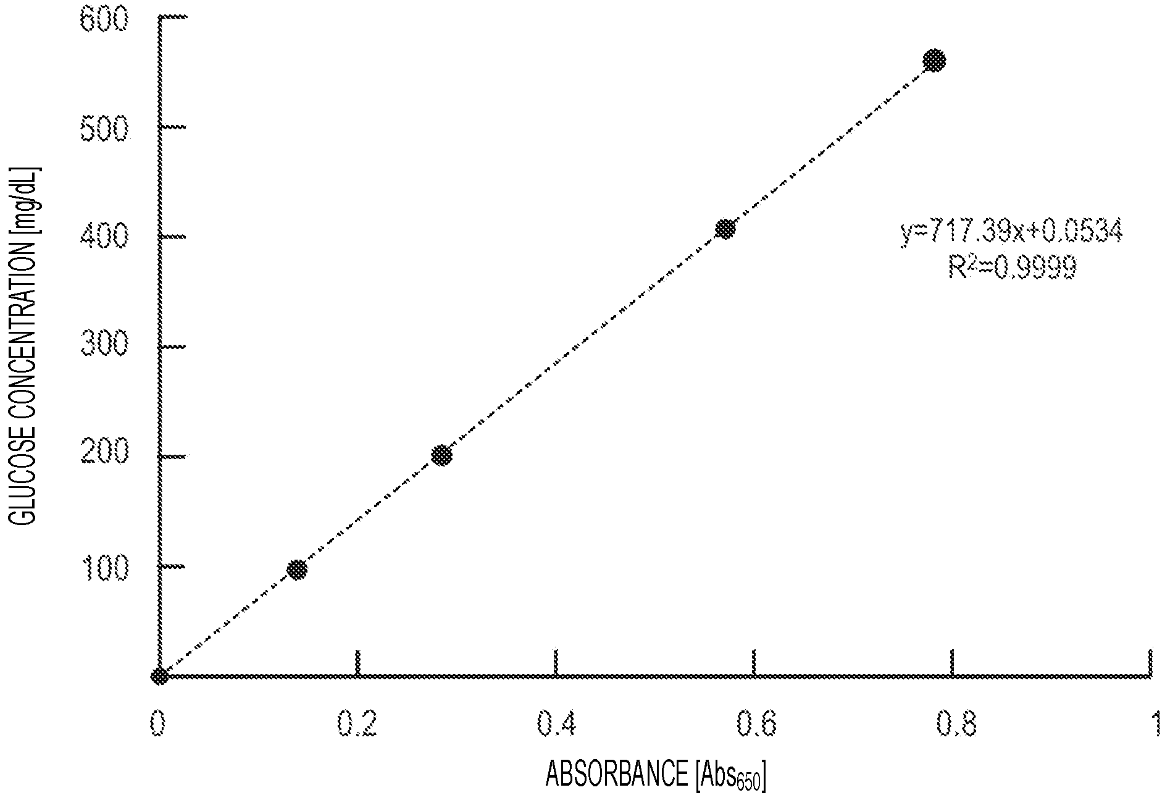
GLUCOSE CONCENTRATION [mg/dL]
600
500
400
300
200
100
0
0.2
0.4
0.6
0.8
1
ABSORBANCE [Abs650]
y=717.39x+0.0534
R2=0.9999

## INTERNATIONAL SEARCH REPORT

International application No.
**PCT/JP2022/037473**

### A. CLASSIFICATION OF SUBJECT MATTER

***G01N 33/52***(2006.01)i; ***C12Q 1/32***(2006.01)i; ***G01N 21/78***(2006.01)i
FI: G01N33/52 B; G01N21/78 Z; C12Q1/32

According to International Patent Classification (IPC) or to both national classification and IPC

### B. FIELDS SEARCHED

Minimum documentation searched (classification system followed by classification symbols)

G01N33/52; C12Q1/26-1/32; G01N21/78

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2019/171731 A1 (TERUMO CORP) 12 September 2019 (2019-09-12) entire text, all drawings | 1-10 |
| A | WO 2018/061484 A1 (TERUMO CORP) 05 April 2018 (2018-04-05) entire text, all drawings | 1-10 |
| A | JP 2003-521246 A (LIFESCAN, INC) 15 July 2003 (2003-07-15) entire text, all drawings | 1-10 |
| A | JP 2015-119729 A (SEKISUI MEDICAL CO LTD) 02 July 2015 (2015-07-02) entire text, all drawings | 1-10 |
| A | JP 2001-292795 A (LIFESCAN, INC) 23 October 2001 (2001-10-23) entire text, all drawings | 1-10 |
| A | WO 2002/027331 A1 (ARKRAY, INC) 04 April 2002 (2002-04-04) entire text, all drawings | 1-10 |
| A | JP 2015-509361 A (I-SENS, INC.) 30 March 2015 (2015-03-30) entire text, all drawings | 1-10 |

☑ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 November 2022** | **22 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**

International application No.
**PCT/JP2022/037473**

**C. DOCUMENTS CONSIDERED TO BE RELEVANT**

| Category* | Citation of document, with indication, where appropriate, of the relevant passages | Relevant to claim No. |
|---|---|---|
| A | US 2021/0025904 A1 (ORTHO-CLINICAL DIAGNOSTICS, INC.) 28 January 2021 (2021-01-28)<br>entire text, all drawings | 1-10 |
| A | US 5077199 A (A. MENARINI S.A.S.) 31 December 1991 (1991-12-31)<br>entire text, all drawings | 1-10 |
| A | CN 112710656 A (BIOSINO BIO-TECHNOLOGY AND SCIENCE INC.) 27 April 2021 (2021-04-27)<br>entire text, all drawings | 1-10 |

Form PCT/ISA/210 (second sheet) (January 2015)

**INTERNATIONAL SEARCH REPORT**
**Information on patent family members**

International application No.
**PCT/JP2022/037473**

| Patent document cited in search report | Publication date (day/month/year) | Patent family member(s) | Publication date (day/month/year) |
|---|---|---|---|
| WO 2019/171731 A1 | 12 September 2019 | US 2020/0371020 A1<br>whole document<br>EP 3736571 A1<br>CN 111788472 A | |
| WO 2018/061484 A1 | 05 April 2018 | US 2019/0178806 A1<br>whole document<br>EP 3521826 A1<br>CN 109477837 A | |
| JP 2003-521246 A | 15 July 2003 | US 2002/0192733 A1<br>whole document<br>WO 2001/057239 A2<br>EP 1252515 A2<br>CA 2388283 A1<br>CZ 20022945 A3<br>PL 358137 A<br>AU 3299101 A<br>CN 1394281 A<br>KR 10-2002-0073190 A<br>AR 27346 A1<br>MX PA02005798 A<br>RU 2002113055 A<br>HK 1049699 A1<br>MY 133942 A | |
| JP 2015-119729 A | 02 July 2015 | US 2015/0072367 A1<br>whole document<br>WO 2013/147309 A1<br>EP 2832862 A1<br>CN 104245952 A<br>KR 10-2014-0142716 A | |
| JP 2001-292795 A | 23 October 2001 | US 6656697 B1<br>whole document<br>EP 1130111 A2<br>AR 027544 A1<br>MY 134005 A<br>BR 0102467 A<br>IL 128905 A<br>ZA 200101543 B<br>AU 2321001 A<br>HK 1038772 A1<br>CA 2337562 A1<br>TW 558638 B<br>RU 2269784 C2<br>KR 10-2001-0085564 A<br>CN 1317575 A<br>MX PA01002062 A<br>SG 100621 A<br>JP 2000-93199 A<br>JP 2000-93198 A | |
| WO 2002/027331 A1 | 04 April 2002 | US 2004/0063213 A1<br>whole document<br>EP 1329722 A1 | |

Form PCT/ISA/210 (patent family annex) (January 2015)

**INTERNATIONAL SEARCH REPORT**
**Information on patent family members**

International application No.
**PCT/JP2022/037473**

| Patent document cited in search report | Publication date (day/month/year) | Patent family member(s) | Publication date (day/month/year) |
|---|---|---|---|
| | | AU 9227901 A | |
| | | CN 1466683 A | |
| JP 2015-509361 A | 30 March 2015 | US 2014/0370539 A1<br>whole document | |
| | | WO 2013/118960 A1 | |
| | | EP 2813854 A1 | |
| | | KR 10-1207418 B1 | |
| | | CN 104105968 A | |
| US 2021/0025904 A1 | 28 January 2021 | WO 2021/016380 A1<br>whole document | |
| | | EP 4003169 A1 | |
| | | CA 3147893 A1 | |
| US 5077199 A | 31 December 1991 | GB 2154735 A<br>whole document | |
| | | DE 3502200 A1 | |
| | | FR 2558849 A1 | |
| | | BE 901570 A | |
| | | CH 664160 A5 | |
| | | IT 1177513 B | |
| | | NL 8500181 A | |
| | | ES 8607554 A1 | |
| | | CA 1263934 A | |
| | | LU 85734 A1 | |
| | | AT 20785 A | |
| | | GR 850181 B | |
| CN 112710656 A | 27 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2000093198 A
- US 5902731 A
- WO 2018051822 A
- WO 2016051930 A
- WO 201851822 A
- WO 2020137532 A
- JP 2021201646 A